# EUROPEAN PATENT SPECIFICATION

(11) **EP 1 933 147 B1**
(45) Date of publication and mention of the grant of the patent: **17.08.2011**
(21) Application number: 06025530.4
(22) Date of filing: 11.12.2006
(51) Int. Cl.: G01N 33/558, G01N 33/543, G01N 33/58, C12Q 1/68

(54) **Rapid immunochromatographic detection by amplification of the colloidal gold signal**
Schnelle immunochromatographische Detektion durch Verstärkung des kolloidalen Gold Signals
Détection rapide immunochromatographique par amplification du signal d'or colloïdal

(43) Date of publication of application: 18.06.2008
(73) Proprietor: AraGen Biotechnology Co. Ltd., 11710 Naor (JO)
(72) Inventor: Badwan, Adnan, Dr., 11185 Amman (JO); Murshed, Abedel-qader Mohammed, 11118 Amman (JO)
(74) Representative: Winkler, Andreas Fritz Ernst

(56) References cited:
- EP-A- 0 590 695
- WO-A-02/46472
- WO-A-2006/039542
- GB-A- 2 284 479
- TANAKA R ET AL: "A novel enhancement assay for immunochromatographic test strips using gold nanoparticles." ANALYTICAL AND BIOANALYTICAL CHEMISTRY, vol. 385, no. 8, August 2006 (2006-08), pages 1414-1420, XP002424110
- GIFFIN B F ET AL: "ENHANCEMENT OF ANTIGENIC SITE DETECTION WITH GOLD LABELED SECONDARY AND TERTIARY ANTIBODIES USING THE IMMUNOGOLD-SILVER STAINING METHOD" BIOTECHNIC AND HISTOCHEMISTRY, vol. 68, no. 6, November 1993 (1993-11), pages 309-315, XP008071261
- OKU Y ET AL: "Development of oligonucleotide lateral-flow immunoassay for multi-parameter detection" JOURNAL OF IMMUNOLOGICAL METHODS, vol. 258, no. 1-2, 1 December 2001 (2001-12-01), pages 73-84, XP004311918
- SIMS P W ET AL: "IMMUNOPOLYMERASE CHAIN REACTION USING REAL-TIME POLYMERASE CHAIN REACTION FOR DETECTION" ANALYTICAL BIOCHEMISTRY, vol. 281, no. 2, 1 June 2000 (2000-06-01), pages 230-232, XP001147707
- HAZARIKA P ET AL: "Sensitive detection of proteins using difunctional DNA-gold nanoparticles" SMALL, WILEY - VCH VERLAG GMBH & CO. KGAA, DE, vol. 1, no. 8-9, 1 January 2005 (2005-01-01), pages 844-848, XP002407187 ISSN: 1613-6810

## Description

The present invention relates to a method for rapid immunochromatographic detection. More precisely, the present invention relates to a method for rapid immunochromatographic detection of a target in a sample, wherein the target is an antibody and/or an antigen, using different colloidal gold conjugates for sensitivity enhancement by signal amplification. The present invention further refers to rapid immunochromatographic detection devices, to the uses of the method for detecting diseases or specific conditions, and to a method for the manufacture of the devices as well as to a kit which comprises the devices.

### BACKGROUND OF THE INVENTION

In recent years, the *in vitro* diagnostics (IVD) industry has made enormous efforts to develop immunochromatographic tests. Such tests have found applications in both clinical and non-clinical fields (1). A clinical utility of this test format has been shown for more than 150 different analytes, and many of them are targets now of commercially available diagnostic products (3). The wide range of applications for such devices has been reviewed (1, 2).

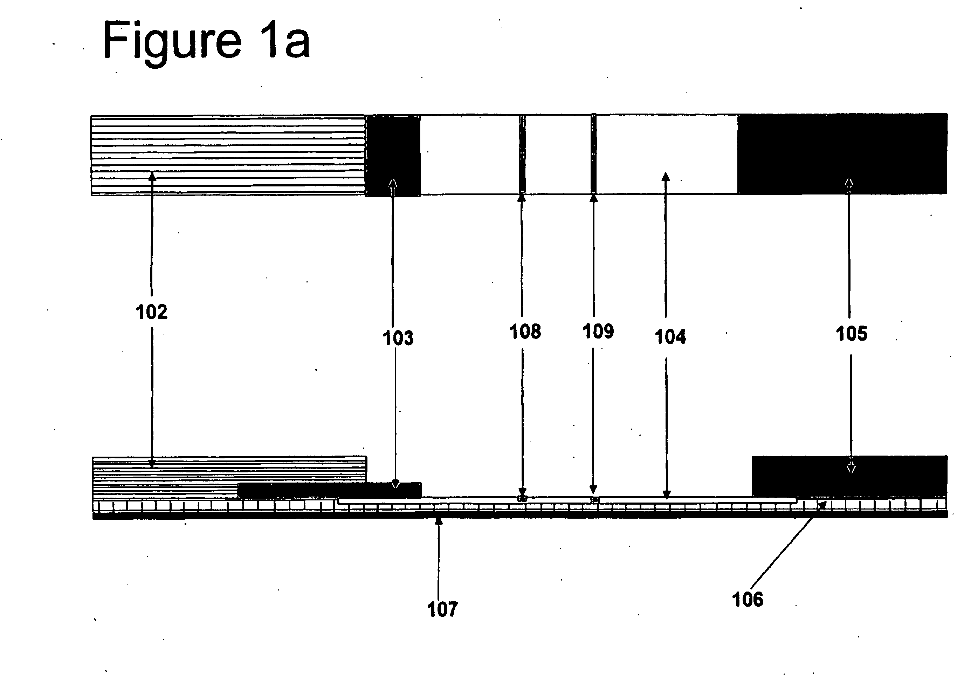

Rapid immunochromatographic test devices, e.g. in the form of a test strip, are made up of a number of components, see Figure 1a. Such a test strip 101 commonly includes a sample pad 102, a conjugate pad 103, a membrane 104, e.g. a nitrocellulose membrane, and an absorbent pad 105. The membrane 104 is usually attached by means of an adhesive 106 to a supporting backing 107, e.g. made of plastic. In practice, the user dispenses a patient sample (usually urine or whole blood) onto the sample pad 102. The sample then flows through the sample pad 102 into the conjugate pad 103, where it mixes with and releases the detector reagent. This mixture then flows across the membrane 104, where it binds with the test and control reagents located in the capture test zone 108 (sample zone) and negative control zone 109, respectively. When the mixture binds to the reagent that forms the test line, a positive result is indicated. The colour intensity of the test line is proportional to the concentration of analyte in the sample. Excess sample that flows beyond the test and control zones 108, 109 is taken up in the absorbent pad 105.

Rapid immunochromatographic test devices for diagnostic purposes are easy to operate and thus do not only contribute to the comfort of professional users, e.g. medical stuff, but also allow the operation by non-professionals users, e.g. most patients.

However, despite the wide use of rapid immunochromatographic test devices, their suitability is still limited with regard to certain applications. Urine, for example, contains very low levels of IgG, frequently around 1 mg/l. Therefore, the detection of antibodies, e.g. directed to HIV or HCV, requires very sensitive techniques. To date, the tests for antibodies in urine samples are based on ELISA and Western blot techniques, which are labour-intensive, time-consuming and need to be carried out by qualified persons. Efforts are being made to develop simple and/or rapid tests for the detection of antibody to HIV in urine specimens (4).

Oral fluid specimens consist often of saliva, which predominantly contains IgA class antibody, and oral mucosal transudates, which mostly contain IgG, and therefore also have much lower levels of IgG than serum. The levels of IgG normally found in oral fluid specimens (approximately 15 mg/l) are, however, higher than in urine specimens and innovative simple and rapid technology that has been shown to be effective for whole blood, serum and plasma, e.g. lateral flow through a chromatographic membrane, has been developed for use with these specimens (4).

Human chorionic gonadotropin (hCG) is a glycopeptide hormone produced by the placenta during pregnancy. The appearance and rapid increase in the concentration of hCG in the subject's urine makes it a good marker for confirming pregnancy. The concentration of hCG in urine increases steadily to a circulation peak of as much as 50,000 mIU/ml between the eighth and eleventh weeks.

Urine hCG levels during pregnancy are estimated to be:
1. 10-30 mIU/ml 7-10 days post conception.
2. 37,000-50,000 mIU/ml 8-11 weeks after last menstrual period.
3. <5 mIU/ml Healthy men or non-pregnant women.

In the prior art the hCG test is a chromatographic immunoassay which uses specific antibodies to selectively identify hCG in urine with a high degree of sensitivity. Elevated levels of hCG as low as 20 mIU/ml can be detected within 3 minutes.
There are several tests used to detect the presence of hepatitis B antibodies. There are also several tests that detect the presence of viral antigens.
The hepatitis B surface antibody (anti-HBs) is the most common test. Its presence indicates previous exposure to HBV, but the virus is no longer present and the person cannot pass on the virus to others. The antibody also protects the body from future HBV infection. In addition to exposure to HBV, the antibodies can also be acquired from successful vaccination. This test is done to determine the need for vaccination (if anti-HBs is absent), or following the completion of vaccination against the disease, or following an active infection.

Hepatitis B surface antigen (HBsAg) is a protein antigen produced by HBV. This antigen is the earliest indicator of acute hepatitis B and frequently identifies infected people before symptoms appear. HBsAg disappears from the blood during the recovery period. In some people (particularly those infected as children or those with a weak immune system, such as those with AIDS), chronic infection with HBV may occur and HBsAg remains positive.
To test for human immunodeficiency virus (HIV) is an essential component in the diagnosis and treatment of persons infected with the virus, in screening of blood for transfusion, in surveillance and in HIV/AIDS related research. Thus accurate and cost-effective testing is of great importance in combating the spread of HIV. It is imperative that tests for the diagnosis of HIV infection be as accurate as possible, given the serious ethical, legal and social issues that accompany HIV infection.

The number of people living with HIV has now risen to reach its highest level ever: close to 40 million people are living with the virus and close to 5 million people were newly infected with HIV in 2004 alone. Worldwide, the AIDS epidemic killed over 3 million people last year alone (Source: UNAIDS). Furthermore, only one in five people needing HIV prevention worldwide have access to basic prevention services and only one in ten people living with HIV has been tested for the virus.

The HI virus is most easily transmitted to others during the initial period of acute HIV infection, when the viral load (quantity of HIV RNA in the blood) is especially high and when people are not aware of being contaminated by the virus. Most HIV infections are transmitted at this stage, called primary infection. Earlier detection using ultra sensitive tests avoids missing primary infections, enabling immediate precautionary measures to be taken to help prevent the risk of HIV transmission to a non-infected partner, to an unborn child, or through blood donations or direct blood contact. Earlier detection of HIV infection also ensures the implementation of early antiretroviral therapy (ART) to slow down the progression of HIV infection, thereby improving patient care and quality of life.

The diagnosis of HIV infection is usually made on the basis of the detection of HIV antibodies and/or antigen. The diagnosis of an HIV infection can be made indirectly, i.e. through the demonstration of virus-specific antibodies. Besides such indirect diagnosis based on detection of antibodies, a direct diagnosis of HIV infection is also possible: either through the demonstration of infectious virus (using cell culture), viral antigens (p24 antigen ELISA) or viral nucleic acid (i.e. viral genome); the latter is also termed nucleic acid testing (NAT).

One important problem of HIV antibody testing is the so-called "diagnostic window". This is the time period that elapses between the times of acquisition of HIV infection until detectable levels of antibodies are present. The switch from antibody-negative to antibody-positive is called "seroconversion".

The most widely used screening tests are ELISAs as they are the most appropriate for screening large numbers of specimens on a daily basis, e.g. blood donations. The earliest assays used purified HIV lysates (1st generation assays). Improved assays based on recombinant proteins and/or synthetic peptides, which also enabled the production of combined HIV-1/HIV-2 assays, became rapidly available (2nd generation assays). The so-called 3rd generation or antigen-sandwich assays, which use labeled antigens as conjugate, are more sensitive and have reduced the diagnostic window period considerably (5, 6).

Tuberculosis (TB) is a major and increasing public health problem in both industrialized and developing countries. Hence, the development of new inexpensive, rapid and field adapted methods for its diagnosis is urgently needed. Sputum culture, which is still the reference method for the diagnosis of pulmonary TB, is cumbersome and time-consuming, and requires access to expensive biosafety level 3 (BSL3) laboratories. Microscopy of direct smears for acid-fast bacilli (AFB) as recommended by WHO for developing countries is the most commonly used method for diagnosis of TB. A major disadvantage with this method is its low sensitivity, even after concentration of the sputum samples.

The availability of new field adapted, low-cost, and rapid diagnostic tests to supplement AFB microscopy, and especially methods improving the diagnosis in AFB-negative disease, would be of great benefit for TB control programs, in particular in areas lacking appropriate safety laboratories. Among the newly developed methods for rapid diagnosis of TB, nucleic acid amplification methods such as PCR seem most promising, but the technology is still too complex to be feasible for TB control programs in developing countries. Antibodies against a number of mycobacterial antigens have been identified in patients using a variety of immunological techniques, but no antibody test has so far reached sufficient sensitivity and/or specificity for routine diagnostic purposes. Detection of circulating or secreted *Mycobacterium tuberculosis* antigens seems attractive and has been explored in a number of studies. However, no satisfactory commercial test for mycobacterial antigens in serum or sputum is currently available.

The idea of identifying mycobacterial antigens in urine of TB patients is attractive for several reasons: urine is more readily obtainable than serum samples and urinary specimens do not carry the risks inherent to needles and blood-based laboratory work. Furthermore, if the urine specimens are boiled before handling, there is no need for BSL3 facilities.

In 1920s, mycobacterial antigens were detected in the urine of TB patients, and the diagnostic potential of such antigens was subsequently discussed by other scientists. More recently, the diagnostic value of mycobacterial antigens in the urine of leprosy patients has been assessed. Unfortunately, the techniques involved turned out to be insufficiently sensitive in paucibacillary patients, the patient group where improved diagnostic tests are most needed.

Lipoarabinomannan (LAM) is a major and structurally important glycolipid component of the outer cell wall of all mycobacteria and may account for up to 15% of the total bacterial weight. LAM is a carbohydrate antigen with glycosidic linkages for which no human degrading glycosidases are known. Hence, we assumed that in active mycobacterial disease LAM may be cleared through the kidneys and occur in urine in antigenically intact form. Furthermore, since LAM is a carbohydrate antigen and thus inherently heat-stable, LAM may be detectable by sensitive immunological techniques, even after boiling of the urine. At least theoretically, the amount of LAM in the urine should reflect the bacterial load, metabolic activity and/or rate of degradation of the bacteria, and hence permit a semi-quantitative assessment of the infectious status. A high sensitive, simple, fast and method for LAM detection and quantification was reported using an enzyme-linked immunosorbent assay (ELISA) in AFB positive sputa from TB patients (7).

EP 0 590 695 refers to a liquid transfer device for use in assay procedures comprising a sheet of porous material for capillary liquid flow therethrough.

GB 2 284 479 refers to a liquid transfer device having utility in diagnostic assays comprising first and second capillary flow channels.

WO 2006/039542 refers to an analytical device for performing an assay to determine the presence or approximate quantity of an analyte in a liquid sample, the device comprising primary and secondary flow paths and a capture zone.

Tanaka *et al.* 2006 (9) refers to a highly sensitive immunochromatographic assay applied to detect hCG as the model case. Primary antibody-conjugated gold nanoparticles were used as an enhancer. The primary antibodies were immobilized within a defined detection zone (test line) on the diagnostic nitrocellulose membrane. Secondary antibodies were conjugated with colloidal gold nanoparticles. The gold-conjugated antibodies and the primary antibodies formed a sandwich complex with the target protein. Within the test line, the sandwich complex was immobilized.

WO 02/46472 refers to the detection of analytes, in particular nucleic acids. The method comprises contacting the nucleic acid with particles having oligonucleotides attached thereto.

Giffin *et al.* 1993 (10) refers to an enhancement of antigenic site detection with gold labeled secondary and tertiary antibodies using the immunogold-silver staining method.

Hazarika *et al.* 2005 (11) refers to the sensitive detection of proteins using an alternative approach based on difunctional DNA-gold nanoparticles.

It is an object of the present invention to overcome the problems with regard to the applicability of rapid immunochromatographic test devices for the detection of hCG, anti-lipoarabinomannan (LAM), HBsAG, anti-HBs, IgG, e.g. HIV antibodies, in urine, blood, serum or saliva by enhanced sensitivity.

### SUMMARY OF THE PRESENT INVENTION

It is an object of the present invention to solve the rapid immunochromatographic detection of a target in a sample by a new method especially through sensitivity enhancement and to overcome the disadvantages of the prior art.

In one embodiment the present invention concerns a method for rapid immunochromatographic detection of a target in a sample comprising the step of forming a sandwich by contacting the sample with
(a) a first colloidal gold conjugate conjugated with a specific antibody or specific antigen to capture the target from a target site and at least one oligonucleotide or non-specific antibody or non-specific antigen; followed by
(b) another specific antibody or specific antigen to capture the target from another target site, wherein the other specific antibody or specific antigen is immobilized; and followed by
(c) a second colloidal gold conjugate conjugated with at least one oligonucleotide being complementary to the oligonucleotide of the first colloidal gold conjugate to capture the first colloidal conjugate from the oligonucleotide site or a non-specific antibody or non-specific antigen being complementary to the non-specific antibody or non-specific antigen of the first colloidal gold conjugate to capture the first colloidal conjugate from a non-specific antibody or non-specific antigen site.

Surprisingly, the object is achieved by the inventive method through signal amplification using different colloidal gold conjugates conjugated with a specific antibody and some oligonucleotides and their complementary oligonucleotides and/or antibodies and their related antigens. The rapid immunochromatographic detection method is using the oligonucleotides to multiply the colloidal gold signal by branched links between both colloidal gold conjugates.

In one embodiment the present invention further relates to a method, comprising the following additional steps of
(d) applying the sample to a sample application site,
(e) allowing the target in the sample getting captured from a target site by the first colloidal gold conjugate at a first conjugate releasing site,
(f) allowing the target in the sample to move to a test zone getting captured from another target site by another immobilized specific antibody or antigen,
(g) allowing to release the second colloidal gold conjugate from a second conjugate releasing site for capturing the first colloidal gold conjugate from at least one oligonucleotide site or non-specific antibody or non-specific antigen site,
(h) allowing the sample to move through the test zone and a control zone to an absorbent site,
(i) allowing to continuously release the first and the second colloidal gold conjugates from the first and the second conjugate releasing sites to propagate to the test zone and the control zone,
(j) detecting a color in the control zone, and
(k) detecting a color in the test zone.

In a further embodiment the present invention concerns a test device for conducting the method for rapid immunochromatographic detection of a target in a sample according to the present invention comprising a housing comprising a test strip (101) comprising
(a) a sample application site (102);
(b) a first conjugate releasing site (103.1) comprising a first colloidal gold conjugate conjugated with a specific antibody or specific antigen to capture the target from a target site and at least one oligonucleotide or non-specific antibody or non-specific antigen;
(c) a second conjugate releasing site (103.2) comprising a second colloidal gold conjugate conjugated with at least one oligonucleotide being complementary to the oligonucleotide of the first colloidal conjugate to capture the first colloidal conjugate from the oligonucleotide site or a non-specific antibody or non-specific antigen being complementary to the non-specific antibody or non-specific antigen of the first colloidal gold conjugate to capture the first colloidal conjugate from a non-specific antibody or non-specific antigen site;
   wherein the first and the second conjugate releasing sites are separated by a divider or wherein the first conjugate releasing site is on the test strip and the second conjugate releasing site is within the upper side of the housing;
(d) a nitrocellulose membrane (104);
(e) a test zone (108) comprising another specific antibody or specific antigen immobilized to capture the target from another target site;
(f) a control zone (109); and
(g) a sample absorbent site (105).

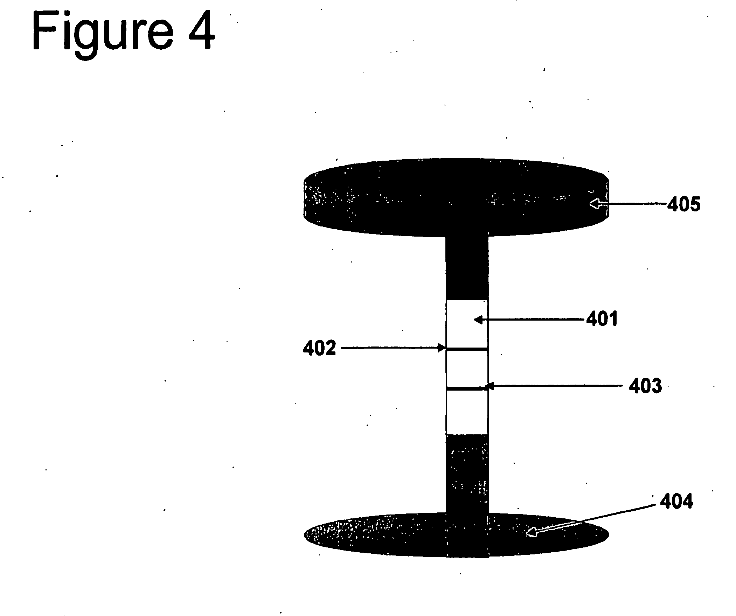

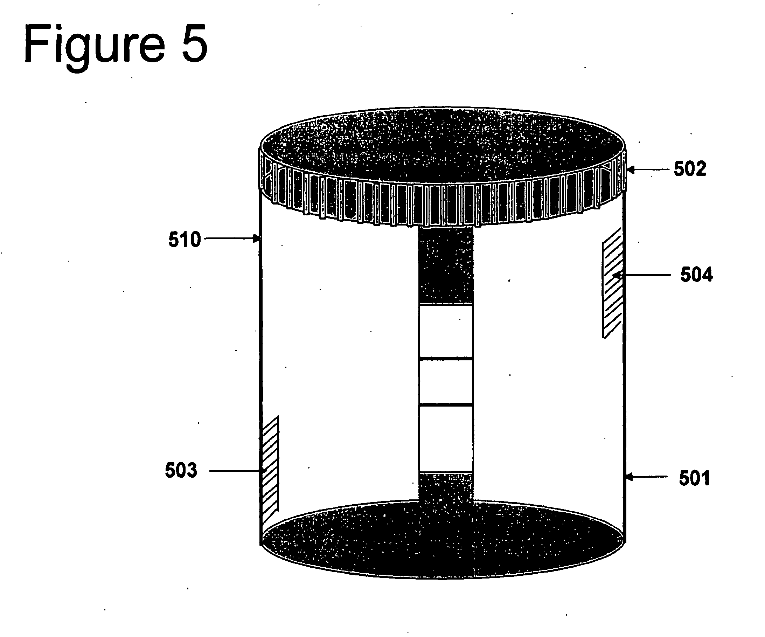

In another embodiment the present invention concerns another test device for conducting the method for rapid immunochromatographic detection of a target in a sample comprising a detection cup (510), see Figure 4 and Figure 5, comprising
(a) a sample collecting container (501)
(b) a test strip comprising a sample application site (404); a nitrocellulose membrane (401); two test zones (403), wherein the test zones (403) comprise another specific antibody or antigen to capture the target from another target site, and a control zone (402); a sample absorbent site (405); and
(c) a first conjugate releasing site (503) comprising a first colloidal gold conjugate conjugated with a specific antibody or specific antigen to capture the target from a target site and at least one oligonucleotide or non-specific antibody or non-specific antigen, and a second conjugate releasing site (504) comprising a second colloidal gold conjugate conjugated with at least one oligonucleotide being complimentary to the oligonucleotide of the first colloidal conjugate to capture the first colloidal conjugate from the oligonucleotide site or a non-specific antibody or non-specific antigen being complementary to the non-specific antibody or non-specific antigen of the first colloidal gold conjugate to capture the first colloidal conjugate from a non-specific antibody or non-specific antigen site, separated from the test strip.

In another embodiment the present invention relates to the use of the method for diagnosing and monitoring a disease or a specific condition of a subject by detecting a target in a sample.

In a further embodiment the present invention refers to a kit for rapid immunochromatographic detection of a target in a sample comprising the test devices according to the present invention, reagents, wash buffers and a manual.

In a further embodiment the present invention concerns a method for the manufacture of the test device according to the present invention comprising the following steps of
(a) preparing a first colloidal gold conjugate by adding a specific antibody or specific antigen to a conjugation buffer with at least one oligonucleotide or non-specific antibody or non-specific antigen and then adding it to a colloidal gold solution;
(b) preparing a second colloidal gold conjugate by adding at least one oligonucleotide being complementary to the oligonucleotide of the first colloidal gold conjugate or a non-specific antibody or non-specific antigen being complementary to the non-specific antibody or non-specific antigen of the first colloidal gold conjugate in a conjugation buffer to a colloidal gold solution; and
(c) preparing a first conjugate releasing site and a second conjugate releasing site by applying the first and the second gold conjugate on different pads;
   wherein the first and second conjugate releasing sites are separated by a divider, or wherein the first conjugate releasing site is on the test strip and the second conjugate releasing site is within the upper side of a housing, or
   wherein the first and second conjugate releasing sites are separated from the test strip in a detection cup.

In another embodiment the method for the manufacture of the test device according to the present invention can additionally comprise the following steps of
(d) preparing a sample application site, a test zone, a control zone and a sample absorbent site,
(e) assembling the sample application site, the test zone, the control zone, the sample absorbent site together with the first and the second conjugate releasing sites on a test strip,
(f) applying the first and the second gold conjugates on different sites of the same card separated by a divider, and
(g) assembling the test strip in a housing.

In a further embodiment the method for the manufacture of the test device according to the present invention can additionally comprise the following steps of
(d) preparing a sample application site, a test zone, a control zone and a sample absorbent site,
(e) assembling the sample application site, the test zone, the control zone, the sample absorbent site together with the first conjugate releasing site on a test strip, and
(f) assembling the test strip and the second conjugate releasing site in a housing.

In another embodiment the method for the manufacture of the test device according to the present invention can additionally comprise the following steps of
(d) preparing a sample application site, two test zones, a control zone and a sample absorbent site,
(e) assembling the sample application site, the test zones, the control zone, and the sample absorbent site on a test strip, and
(f) assembling the test strip and the first and second conjugate releasing sites separately in a detection cup.

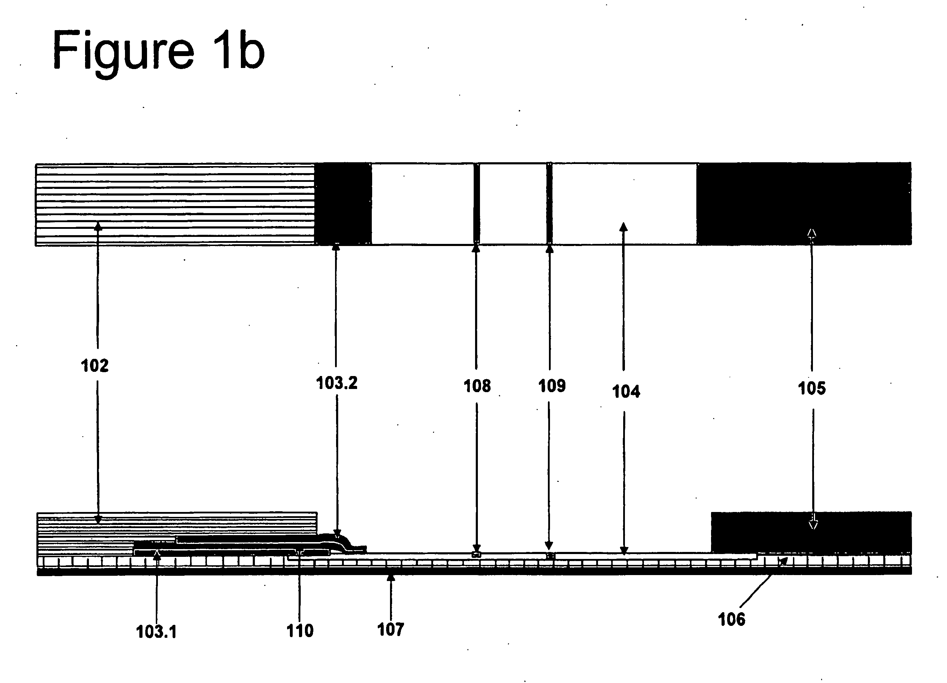

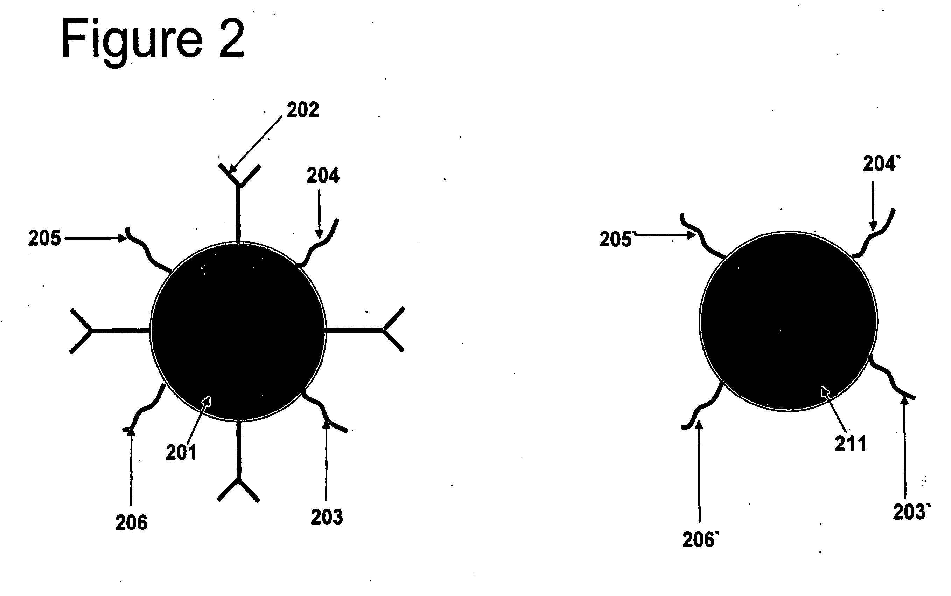

The rapid immunochromatographic detection system comprises a detection test strip of two gold conjugate releasing sites or pads with different compositions. The first conjugate releasing site or pad 103.1 is laminated on the test strip between the sample application site or sample pad and the nitrocellulose membrane, while the second conjugate releasing site or pad 103.2 is laminated above the first conjugate releasing site separated by a divider 110 to be released directly toward the nitrocellulose membrane without flow through the first conjugate releasing site to avoid interaction with the first conjugate before reaching the membrane, see Figure 1b. The second conjugate releasing site can also be laminated within the upper side of the plastic housing of the device. The first conjugate releasing pad contains a gold that is conjugated with the detection specific antibody/antigen and (at the same time) with some specific oligonucleotides, while the second conjugate releasing pad contains a gold conjugated with the complementary oligonucleotides that are conjugated with the first colloidal gold conjugate, see Figure 2. Specific antibodies/antigens which should differ from the detection specific antibodies/antigens with their specific antigens/antibodies may be employed to play the same role as the oligonucleotides and their complementary oligonucleotides. The first conjugate releasing pad is containing an extra amount of Tween 20 that enables the first conjugate to be released before the second conjugate.

By the sample flow within the rapid immunochromatographic test the antibody on the first pad which contains the oligonucleotides will capture the antigen/antibody in the sample and carry it to be captured by the other antibody/antigen that is immobilized on the nitrocellulose membrane to form the sandwich detection. Then, the second conjugate releasing pad will release its gold that is conjugated with the complementary oligonucleotides. The last mentioned conjugate would bind with the first conjugate from the oligonucleotide(s) side. This binding could be happened by any of the conjugated oligonucleotides with its complementary oligonucleotide on the other gold conjugate. At the same time, the other oligonucleotides will be able to link with their complementary oligonucleotides besides the probability of capturing the first conjugate that will capture the second conjugate to form more and more branched bonds which propagates the accumulation of colloidal gold particles onto the capturing/sample line. This propagation and accumulation of colloidal gold signal will amplify the signal and highly increase the sensitivity. This will enable us to detect very low concentrations that are not detectable using the same technique without signal amplification.

### DETAILED DESCRIPTION OF THE INVENTION

Before the present invention is described in more detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art. For the purposes of the present invention, all references as cited herein are incorporated by reference in their entireties.

Preferably, the terms used herein are defined as described in "A multilingual glossary of biotechnological terms: (IUPAC Recommendations)", Leuenberger, H.G.W., Nagel, B. and Kölbl. H. eds. (1996), Helvetica Chimica Acta, CH-4010 Basel, Switzerland).

Throughout this specification and the claims which follow, unless the context requires otherwise, the word "comprise", and variations such as "comprises" and "comprising", will be understood to imply the inclusion of a stated integer or step or group of integers or steps, but not the exclusion of any other integer or step or group of integer or step.

As outlined above there is a need in the prior art to provide a new method for rapid immunochromatographic detection of a target in a sample for the detection of a disease or a specific condition such as pregnancy in a subject. There is also a need in the art for methods suitable for rapid and sensitive detection of an antibody and/or antigen having a higher sensitivity than methods from the prior art.

In a first aspect the present invention provides a method for rapid immunochromatographic detection of a target in a sample comprising the step of forming a sandwich by contacting the sample with
(a) a first colloidal gold conjugate conjugated with a specific antibody or specific antigen to capture the target from a target site and at least one oligonucleotide or non-specific antibody or non-specific antigen; followed by
(b) another specific antibody or specific antigen to capture the target from another target site, wherein the other specific antibody or specific antigen is immobilized; and followed by
(c) a second colloidal gold conjugate conjugated with at least one oligonucleotide being complementary to the oligonucleotide of the first colloidal gold conjugate to capture the first colloidal conjugate from the oligonucleotide site or a non-specific antibody or non-specific antigen being complementary to the non-specific antibody or non-specific antigen of the first colloidal gold conjugate to capture the first colloidal conjugate from a non-specific antibody or non-specific antigen site.

The first colloidal gold conjugated with a first antibody or antigen captures the target in the sample and forms a complex "target-first colloidal conjugate". Preferably this target in the sample is an antigen and/or antibody.

In a preferred embodiment of the method according to the present invention each gold conjugate comprises between 1 and 15 different oligonucleotides, e.g. 1, 2, 3, 4, 5, 6, 7, 8, 9, 10, 11, 12, 13, 14, 15, preferably between 2 and 10 different oligonucleotides, more preferably between 3 and 6 different oligonucleotides. In addition, the oligonucleotides conjugated with the second gold conjugate are complementary to the oligonucleotides of the first colloidal gold conjugate.

Mostly 2-4 oligonucleotides per gold conjugate are used. These oligonucleotides are of about 20-nucleotides in length. These oligonucleotides have an amino group at the 5' terminus which is conjugated with bovine serum albumin. The bond between the gold and the oligonucleotides is the same as the one between the gold and the antibodies or antigens.

In a further first aspect the present invention provides a method comprising the following additional steps of
(d) applying the sample to a sample application site,
(e) allowing the target in the sample getting captured from a target site by the first colloidal gold conjugate at a first conjugate releasing site,
(f) allowing the target in the sample to move to a test zone getting captured from another target site by another immobilized specific antibody or antigen ,
(g) allowing to release the second colloidal gold conjugate from a second conjugate releasing site for capturing the first colloidal gold conjugate from at least one oligonucleotide site or non-specific antibody or non-specific antigen site,
(h) allowing the sample to move through the test zone and a control zone to an absorbent site,
(i) allowing to continuously release the first and the second colloidal gold conjugates from the first and the second conjugate releasing sites to propagate to the test zone and the control zone,
(j) detecting a color in the control zone, and
(k) detecting a color in the test zone.

In one embodiment the method comprises further a first specific antibody or antigen which is selected from the group consisting of anti-beta chorionic gonadotropin hormone (anti-βhCG), anti-lipoarabinomannan (LAM), hepatitis virus antibodies against or antigens from hepatitis virus type A , hepatitis virus type B, or hepatitis virus type C or human immunoglobulin G antibodies or antigens.

Other antibodies and antigens which can be used are HIV specific antibodies or antigens, tuberculosis specific antibodies or antigens, malaria specific antibodies, toxoplasmosis specific antibodies or antigens, rubella specific antibodies, Leishmania specific antibodies or Pneumonia specific antibodies. Monoclonal antibodies are preferred, whereas polyclonal antibodies are applicable.

In one preferred embodiment the hepatitis virus antigen is hepatitis B surface antigen (HBsAg) and the hepatitis virus antibody is anti-HBsAg.

In one embodiment the method comprises further another specific antibody or antigen which is selected from the group consisting of anti-alpha chorionic gonadotropin hormone (anti-αhCG), anti-lipoarabinomannan (LAM), hepatitis virus antibodies against or antigens from hepatitis virus type A, hepatitis virus type B, or hepatitis virus type C or human immunodeficiency virus (HIV) antibodies or antigens from the HIV type HIV-1 and HIV-2 or HIV subtype HIV-1-N, HIV-1-O or HIV-1-M.

In one preferred embodiment the hepatitis virus antigen is hepatitis B surface antigen (HBsAg), the hepatitis virus antibody is anti-HBsAg and the human immunodeficiency virus (HIV) antigen is HIV p160.

Moreover, antigen detection antibodies are often pairs of monoclonal antibodies for example of anti-hepatitis B surface antigen (anti-HBsAg), anti-HIV p160 or anti-HIV p24.

In one embodiment of the method the sample comprises a body fluid obtained from a subject.

In one preferred embodiment the body fluid is selected from the group consisting of urine, whole blood, serum, plasma and saliva.

In another aspect the present invention concerns a test device for conducting the method for rapid immunochromatographic detection of a target in a sample according to the present invention comprising a housing comprising a test strip 101 comprising
(a) a sample application site (102);
(b) a first conjugate releasing site (103.1) comprising a first colloidal gold conjugate conjugated with a specific antibody or specific antigen to capture the target from a target site and at least one oligonucleotide or non-specific antibody or non-specific antigen;
(c) a second conjugate releasing site (103.2) comprising a second colloidal gold conjugate conjugated with at least one oligonucleotide being complementary to the oligonucleotide of the first colloidal conjugate to capture the first colloidal conjugate from the oligonucleotide site or a non-specific antibody or non-specific antigen being complementary to the non-specific antibody or non-specific antigen of the first colloidal gold conjugate to capture the first colloidal conjugate from a non-specific antibody or non-specific antigen site;
   wherein the first and the second conjugate releasing sites are separated by a divider or wherein the first conjugate releasing site is on the test strip and the second conjugate releasing site is within the upper side of the housing;
(d) a nitrocellulose membrane (104);
(e) a test zone (108) comprising another specific antibody or specific antigen immobilized to capture the target from another target site;
(f) a control zone (109); and
(g) a sample absorbent site (105).

The first conjugate releasing site or pad 103.1 is laminated on the test strip between the sample pad and the nitrocellulose membrane, while the second conjugate releasing site or pad 103.2 is located above the first conjugate releasing pad separated by a divider 110, in order to be released directly toward the nitrocellulose membrane without flow through the first conjugate pad to avoid interact with the first conjugate before reaching the membrane, see Figure 1b. The second conjugate releasing site can be laminated within the upper side of the plastic housing of the device.

In one embodiment the test device further comprises the test strip 101 which is attached to a supporting backing 107 by means of an adhesive 106.

In a preferred embodiment the supporting backing 107 of the test device is a plastic backing.

In another embodiment the test zone 108 of the test device comprises another specific antibody or antigen.

In another preferred embodiment the test zone 108 of the test device comprises another specific antibody or antigen which is selected from the group consisting of anti-alpha chorionic gonadotropin hormone (anti-αhCG), anti-lipoarabinomannan (LAM), hepatitis virus antibodies against or antigens from hepatitis virus type A, hepatitis virus type B, or hepatitis virus type C or human immunodeficiency virus (HIV) antibodies or antigens from the HIV type HIV-1 and HIV-2 or HIV subtype HIV-1-N, HIV-1-O or HIV-1-M.

In a more preferred embodiment the hepatitis virus antigen is hepatitis B surface antigen (HBsAg), the hepatitis virus antibody is anti-HBsAg and the human immunodeficiency virus (HIV) antigen is HIV p160.

Moreover, antigen detection antibodies are often pairs of monoclonal antibodies for example of anti-hepatitis B surface antigen (anti-HBsAg), anti-HIV p160 or anti-HIV p24.

In another embodiment of the present invention the second conjugate releasing site 103.2 is laminated within the upper side of the housing.

In a further aspect the present invention concerns a test device for conducting the method for rapid immunochromatographic detection of a target in a sample according to the present invention comprising a detection cup 510 comprising
(a) a sample collecting container (501)
(b) a test strip comprising a sample application site (404); a nitrocellulose membrane (401); two test zones (403), wherein the test zones (403) comprise another specific antibody or antigen to capture the target from another target site, and a control zone (402); a sample absorbent site (405); and
(c) a first conjugate releasing site (503) comprising a first colloidal gold conjugate conjugated with a specific antibody or specific antigen to capture the target from a target site and at least one oligonucleotide or non-specific antibody or non-specific antigen, and a second conjugate releasing site (504) comprising a second colloidal gold conjugate conjugated with at least one oligonucleotide being complimentary to the oligonucleotide of the first colloidal conjugate to capture the first colloidal conjugate from the oligonucleotide site or a non-specific antibody or non-specific antigen being complementary to the non-specific antibody or non-specific antigen of the first colloidal gold conjugate to capture the first colloidal conjugate from a non-specific antibody or non-specific antigen site, separated from the test strip.

In one embodiment the test zones of the detection cup further comprise another specific antibody or antigen.

In a preferred embodiment another specific antibody or antigen is selected from the group consisting of anti-alpha chorionic gonadotropin hormone (anti-αhCG), anti-lipoarabinomannan (LAM), hepatitis virus antibodies against or antigens from hepatitis virus type A , hepatitis virus type B, or hepatitis virus type C or human immunodeficiency virus (HIV) antibodies or antigens from the HIV type HIV-1 and HIV-2 or HIV subtype HIV-1-N, HIV-1-O or HIV-1-M.

In one preferred embodiment the hepatitis virus antigen is hepatitis B surface antigen (HBsAg), the hepatitis virus antibody is anti-HBsAg and the human immunodeficiency virus (HIV) antigen is HIV p160.

Moreover, antigen detection antibodies are often pairs of monoclonal antibodies for example of anti-hepatitis B surface antigen (anti-HBsAg), anti-HIV p160 or anti-HIV p24.

In another aspect the invention relates to the use of the method for diagnosing and monitoring a disease or a specific condition of a subject by detecting a target in a sample.

In one embodiment the specific condition is pregnancy.

In a preferred embodiment the target of the specific condition is human chorionic gonadotropin hormone (hCG).

In another embodiment the disease is hepatitis selected of the group consisting of hepatitis type A, hepatitis type B, or hepatitis type C.

In a preferred embodiment the selected hepatitis type is hepatitis type B.

In a more preferred embodiment the target of the disease which is hepatitis type is hepatitis B surface antigen (HBsAg).

In another embodiment the disease is an HIV infection selected from the HIV infection group consisting of HIV type HIV-1 and HIV-2 or HIV subtype HIV-1-N, HIV-1-O or HIV-1-M.

In a more preferred embodiment the target of the HIV infection is selected from an HIV antibody or antigen selected from the group consisting of p41, p120, p160, p18, p24/25, p55, p34, p40, p52, p68.

In a further more preferred embodiment the HIV antigen is p160.

In a further aspect the invention concerns a kit for rapid immunochromatographic detection of a target in a sample comprising the test device comprising the housing according to the invention.

In one embodiment the kit comprises further reagents, wash buffers and a manual.

In another aspect the invention relates to a method for the manufacture of the test device according to the invention

comprising the following steps of
(a) preparing a first colloidal gold conjugate by adding a specific antibody or specific antigen to a conjugation buffer with at least one oligonucleotide or non-specific antibody or non-specific antigen and then adding it to a colloidal gold solution;
(b) preparing a second colloidal gold conjugate by adding at least one oligonucleotide being complementary to the oligonucleotide of the first colloidal gold conjugate or a non-specific antibody or non-specific antigen being complementary to the non-specific antibody or non-specific antigen of the first colloidal gold conjugate in a conjugation buffer to a colloidal gold solution; and
(c) preparing a first conjugate releasing site and a second conjugate releasing site by applying the first and the second gold conjugate on different pads;
   wherein the first and second conjugate releasing sites are separated by a divider, or wherein the first conjugate releasing site is on the test strip and the second conjugate releasing site is within the upper side of a housing, or
   wherein the first and second conjugate releasing sites are separated from the test strip in a detection cup.

In one embodiment the method further comprises the following steps of
(d) preparing a sample application site 102, a test zone 108, a control zone 109 and a sample absorbent site 105,
(e) assembling the sample application site 102, the test zone 108, the control zone 109, the sample absorbent site 105 together with the first 103.1 and the second conjugate releasing sites on a test strip 101,
(f) applying the first and the second gold conjugates on different sites of the same card separated by a divider 110, and
(g) assembling the test strip 101 in a housing.

In another embodiment the method further comprises the following steps of
(d) preparing a sample application site 102, a test zone 108, a control zone 109 and a sample absorbent site 105,
(e) assembling the sample application site 102, the test zone 108, the control zone 109, the sample absorbent site 105 together with the first conjugate releasing site 103.1 on a test strip 101, and
(f) assembling the test strip 101 and the second conjugate releasing site 103.2 in a housing.

In a further embodiment the method further comprises the following steps of
(d) preparing a sample application site 404, two test zones 403, a control zone 402 and a sample absorbent site 405,
(e) assembling the sample application site 404, the test zones 403, the control zone 402, and the sample absorbent site 405 on a test strip, and
(f) assembling the test strip and the first 503 and second conjugate 504 releasing sites separately in a detection cup 510.

### BRIEF DESCRIPTION OF THE FIGURES

**Figure 1a****:**
   Figure 1a shows top and side views of a typical rapid-flow immunochromatographic test device in the form of a test strip 101 including a sample pad 102, a conjugate pad 103, a membrane 104, an absorbent pad 105, an adhesive 106, a supporting backing 107, a test zone 108, and a control zone 109.
**Figure 1b****:**
   Figure 1b shows top and side views of our modified rapid-flow immunochromatographic test device in the form of a test strip 101 including a sample pad 102, a first conjugate pad 103.1, a second conjugate pad 103.2, a membrane 104, an absorbent pad 105, an adhesive 106, a supporting backing 107, a test zone 108, a control zone 109, and the two conjugates divider 110.
**Figure 2****:**
   Figure 2 shows the schematically view of the first and second colloidal gold, whereas the first colloidal gold 101 is conjugated with an antibody 202 and four different oligonucleotides 203, 204, 205, 206 and the second colloidal gold 211 is conjugated with four oligonucleotides 203', 204', 205', 206' complementary to the oligonucleotides of the first colloidal gold 101.
**Figure 3****:**

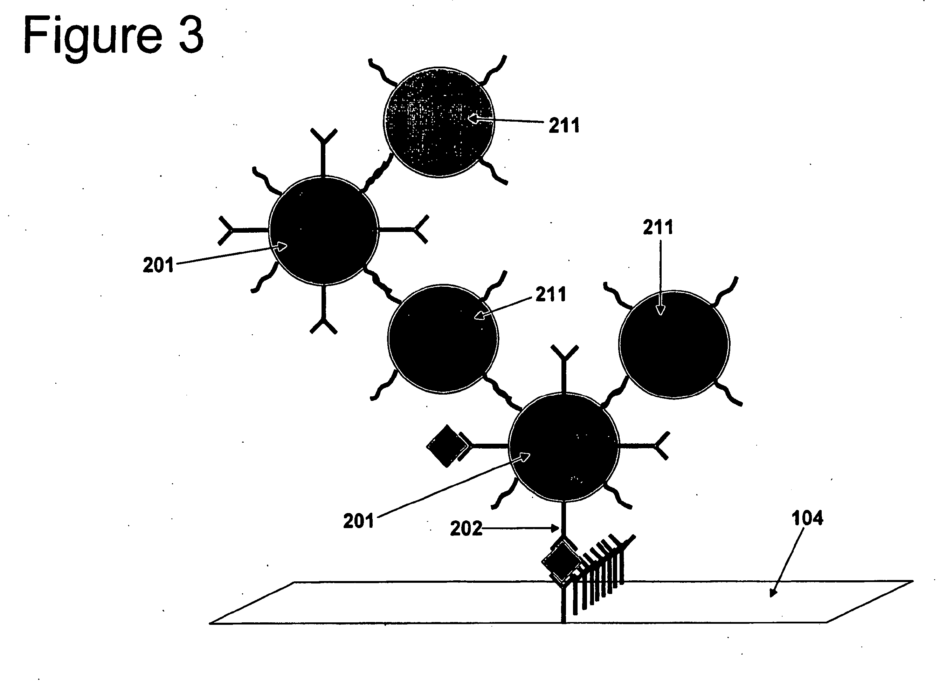

   Figure 3 shows the main principle of signal amplification:
   By the sample flow within the rapid immunochromatographic test the target in the sample 220 will be captured by the antibody or antigen 202 of the first colloidal gold 201 to form the complex "target- first colloidal gold". This complex flows to the test zone, where it will be captured by another antibody or antigen 108 that is immobilized onto the membrane 104 of the test zone to form a sandwich detection. Then, the second colloidal gold 211 conjugated with the complementary oligonucleotides 203', 204', 205', 206' to the oligonucleotides 203, 204, 205, 206 of the first colloidal gold will be released and will bind to the first conjugate from the oligonucleotide(s) side and enhance the signal.
**Figure 4****:**
   Figure 4 shows an assembly comprising a nitrocellulose membrane 401 with a control line 402 and a sample line 403, as well as a sample pad 404 and an absorbent pad 405.
**Figure 5****:**
   Figure 5 shows an immunochromatographic detection cup 510 comprising a sample collecting container 501, a cap 502 that contains the absorbent pad 405 and a similar assembly as shown in Figure 4, except that there are two sample lines 403. Furthermore, there are the first conjugate releasing pad 503 and the second conjugate releasing pad 504 which are separately fixed from each other to the wall of the container.

### EXAMPLES

The following examples illustrate the present invention without, however, limiting the same thereto.

### Example 1 : Preparation of oligonucleotide and complementary oligonucleotide labeled bovine serum albumin

5mg of bovine serum albumin (BSA) was linked to each oligonucleotide (about 20 nucleotide having an amino group at 5' terminus) and another 5mg to complementary oligonucleotide(about 20 nucleotide having an amino group at 5' terminus), according to a procedure comprising the following steps, according to the method described by Duncan et al. 1983 (7):

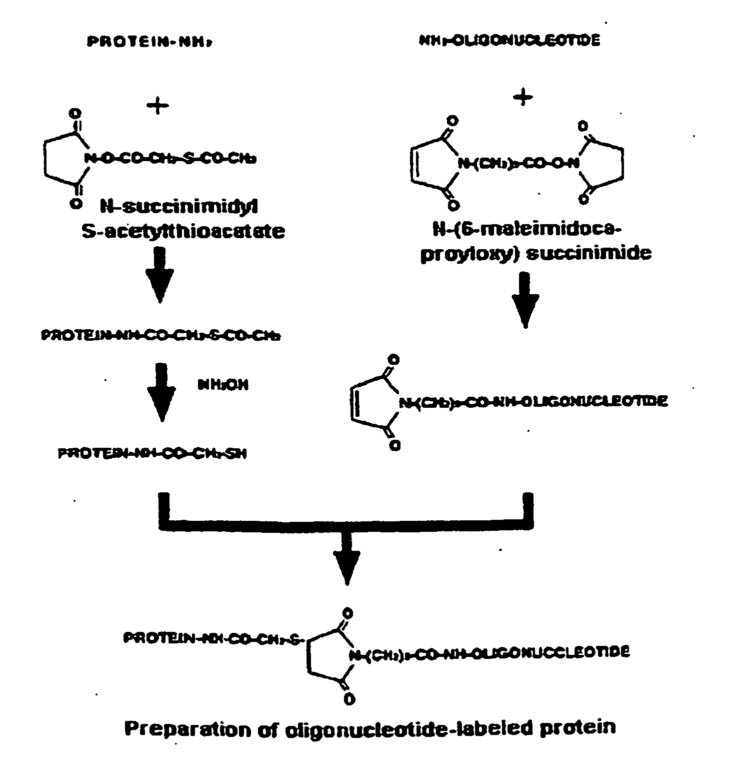

### Example 2: Manufacturing procedure of a test device

The oligonucleotide and complementary oligonucleotide labeled bovine serum albumin (BSA) prepared as described in Example 2 are further processed according to a procedure comprising the following steps:
1. Preparation of oligonucleotides* 203, 204, 205, 206-labeled bovine serum albumin (BSA) (solution 1), see Figure 2, (solution 1).
2. Preparation of complementary oligonucleotides* 203', 204', 205', 206'-labeled bovine serum albumin (solution 2), see Figure 2, (solution 2).
3. Preparation of a 1% aqueous solution of tetrachloroauric acid at room temperature;
4. Preparation of a 4% trisodium citrate aqueous solution at room temperature;
5. Preparation of a 0.05 M potassium carbonate aqueous solution at room temperature;
6. Preparation of 400ml of phosphate stabilizing buffer of pH 7.4 that contains BSA, Tween 20, Sucrose, polyvinylpurrolidone and preservative (like sodium azide) at room temperature;
7. Preparation of a colloidal gold solution by reduction of 1.7 ml boiling tetrachloroauric acid solution (after dilution into 100ml) using 1ml trisodium citrate solution and let it takes the room temperature;
8. Dilution of the colloidal gold solution as 1:1 using distilled water. Adjust the pH to 7.4 using potassium carbonate solution at room temperature;
9. Preparation of 200ml of phosphate conjugation buffer of pH 7.4 at room temperature;
10. Partition of the 200ml conjugation buffer by dividing it into two flasks (100ml of each);
11. Addition of 0.5 mg of aqueous antibody (e.g. anti-hIgG or anti-βhCG) to the conjugation buffer in the first flask with stirring at room temperature;
12. Addition of 0.5 mg of oligonucleotides labelled BSA aqueous solution (solution 1) to the first flask at room temperature;
13. Addition of 1.0 mg of complementary oligonucleotides labelled BSA aqueous solution (solution 2) to the conjugation buffer in the second flask with stirring at room temperature;
14. Addition of 100ml colloidal gold solution into each flask with stirring at room temperature;
15. Addition of 200ml of stabilizing buffer to each flask and concentrate each conjugate by cooled (temperature around 15°C ) high speed centrifugation (10,000 rpm for one hour);
16. Discarding the supernatant and resuspending the concentrated conjugates at room temperature;
17. Adjusting the concentration for each of the two conjugates to O.D.₅₂₀=2.0;
18. Addition of 0.1ml of Tween 20 only to the first conjugate and soaking glass fibre sheet conjugate pad into the conjugate, then heating dry at temperature around 50°C.
19. Soaking another glass fiber sheet conjugate pad into the second conjugate, then heating dry at temperature around 50°C.
20. Printing of sample (e.g. anti-αhCG) and control lines onto the nitrocellulose membranes.
21. Lamination of cards using the first gold conjugate. Lamination of card components onto the backing material with the sequence (in case of conjugate releasing site laminated within the upper side of the device plastic housing):
   a. Laminate the nitrocellulose membrane nearly in the middle of the card.
   b. Laminate the absorbent pad in the end of the card (overlaps from the nitrocellulose membrane side).
   c. Laminate the first conjugate pad in the other side of the nitrocellulose membrane.
   d. Laminate the sample pad.
   Lamination of card components onto the backing material with the sequence (in case of conjugate releasing site laminated onto the test strip itself separated from the first conjugate by a divider), see Figure 1b:
   a. Laminate the nitrocellulose membrane nearly in the middle of the card.
   b. Laminate the absorbent pad in the end of the card (overlaps from the nitrocellulose membrane side).
   c. Laminate the first conjugate pad in the other side of the nitrocellulose membrane.
   d. Laminate the plastic divider onto the first conjugate (overlaps from the nitrocellulose membrane side).
   e. Laminate the second conjugate pad onto the divider (overlaps from the nitrocellulose membrane side).
   f. Laminate the sample pad onto the other end of the card, the sample pad will overlaps with the two conjugate pads.
22. Then cut cards into strips.
23. Lamination of the second gold conjugate will applied within the plastic housing itself to ensure that the two conjugates will not propagate before release from the releasing pad and so stick within the releasing pad.
   The first conjugate releasing pad 103.1 is laminated on the test strip between the sample pad and the nitrocellulose membrane while the second 103.2 is above the first pad separated by a divider 110 to be released directly toward the nitrocellulose membrane without flow through the first conjugate pad to avoid interact with the first conjugate before reaching the membrane, see figure 1b. The second conjugate releasing site could be laminated within the upper side of the device plastic housing.
24. In case of a detection cup as the test device for urine or saliva, the two gold conjugates are fixed in different positions into the internal surface of the cup, for the same reason as in 13, see Figures 5 and 6.
   Finally the lamination of the second gold conjugate will be applied within the plastic housing itself to ensure that the two conjugates will not propagate before release from the releasing pad and so stick within the releasing pad.

* In case of antibodies/antigens and their specific antigens/antibodies there is no need for these steps of bovine serum albumin or any other protein labeling.
* * Other proteins or peptides could be used other than bovine serum albumin

### Example 3: Pregnancy detection system

The first gold conjugate is made of mouse anti-βhCG and four oligonucleotides conjugated with colloidal gold conjugate, and the second gold conjugate is the conjugate of the four complementary oligonucleotides. The first gold conjugate 103.1 was laminated in the side of the nitrocellulose membrane, while the second gold conjugate 103.2 is laminated above the first pad 103.1 separated by a divider 110 that enables the second conjugate to take a part of the sample and release directly onto the nitrocellulose membrane.

The plastic housing is the plastic design where we insert the test strip. The first conjugate releasing pad 103.1 is laminated on the test strip between the sample pad and the nitrocellulose membrane while the second 103.2 is above the first pad separated by a divider 110 to be released directly toward the nitrocellulose membrane without flow through the first conjugate pad to avoid interact with the first conjugate before reaching the membrane, see figure 1b. The second conjugate releasing site could be laminated within the upper side of the device plastic housing.

The sample line is a mouse anti-αhCG immobilized onto the nitrocellulose membrane. The control line is anti-mouse IgG. Sample and control lines turn into purple color in case of hCG availability in the sample; only the control line turns into purple color in case of hCG free sample.
The commercially available rapid tests sensitivity for the pregnancy hormone which is human chorionic gonadotropin hormone (hCG) is around 25mIU/ml while according to this system it is so simple to detect less than 1 mIU/ml.

### Example 4: Hepatitis B surface antigen (HBsAg) detection system

The first gold conjugate is made of mouse anti-HBsAg (clone 1) and four oligonucleotides conjugated with colloidal gold conjugate, and the second gold conjugate is the conjugate of the four complementary oligonucleotides. The numbering of clones are only for explanation and to recognize that we use always two different clones of monoclonal antibodies; these two monoclonal antibodies capture the target antigen from two different sites, so we call them a pair of monoclonal antibodies. The first gold conjugate 103.1 was laminated in the side of nitrocellulose membrane 104, while the second gold conjugate 103.2 was laminated above the first pad 103.1 separated by a divider 110 that enables the second conjugate to take a part of the sample and release it directly onto the nitrocellulose membrane.

The plastic housing is the plastic design, where we insert the test strip. The first conjugate releasing pad 103.1 is laminated on the test strip between the sample pad and the nitrocellulose membrane while the second 103.2 is above the first pad separated by a divider 110 to be released directly toward the nitrocellulose membrane without flow through the first conjugate pad to avoid interact with the first conjugate before reaching the membrane, see Figure 1b. The second conjugate releasing site could be laminated within the upper side of the device plastic housing.

The sample line 108 is mouse anti-HBsAg (clone2) immobilized onto the nitrocellulose membrane 104. The control line 109 is anti-mouse IgG. Sample 108 and control lines 109 turn into purple color in case of HBsAg availability in the sample; only the control line 109 turns into purple color in case of HBsAg free sample, see Figure 1b.

The commercially available rapid tests sensitivity for hepatitis B surface antigen is within the range 500-1000pg/ml while according to this system it is so simple to detect less than 10 pg/ml.

### Example 5: Human Immunodeficiency Virus (HIV) antibodies detection system

The first gold conjugate is mouse anti-human Immunoglobulin G (anti-hIgG) and four oligonucleotides conjugated with colloidal gold conjugate, and the second gold conjugate is the conjugate of the four complementary oligonucleotides. The first gold conjugate 103.1 was laminated in the side of nitrocellulose membrane 104, while the second gold conjugate 103.2 was laminated above the first pad 103.1 separated by a divider that enables the second conjugate to take a part of the sample and release it directly onto the nitrocellulose membrane 104.

The plastic housing is the plastic design where we insert the test strip. The first conjugate releasing pad 103.1 is laminated on the test strip between the sample pad and the nitrocellulose membrane while the second 103.2 is above the first pad separated by a divider 110 to be released directly toward the nitrocellulose membrane without flow through the first conjugate pad to avoid interact with the first conjugate before reaching the membrane, see figure 1b. The second conjugate releasing site could be laminated within the upper side of the device plastic housing.

The sample line 108 is a combination of synthetic or recombinant HIV antigen immobilized onto the nitrocellulose membrane 104. The control line 109 is anti-mouse IgG. Sample 108 and control 109 lines turn into purple color in case of HIV antibodies availability in the sample; only the control line 109 turns into purple color in case of HIV antibodies free sample, see Figure 1b.

According to this system it is so simple to detect very low titers of HIV antibodies in serum.

### References

(1) J Chandler, N Robinson, and K Whiting, "Handling False Signals in Gold-Based Rapid Tests", IVD Technology 7, no. 2 (2001): 34-45; http://www.devicelink.com/ivdt/archive/01/03/002.html.
(2) J Chandler, T Gurmin, and N Robinson, "The Place of Gold in Rapid Tests", IVD Technology 6, no. 2 (2000): 37-49; http://www.devicelink.com/ivdt/archive/00/03/004.html
(3)TC Tisone et al., "Image Analysis for Rapid-Flow Diagnostics", IVD Technology 5, no. 5 (1999): 52-58; http://www.devicelink.com/ivdt/archive/99/09/010.html.
(4) World Health Organization, HIV assays: operational characteristics (Phase I). Report 13: urine specimens, oral fluid (saliva) specimens. [Material originally distributed as WHO/BCT/02.08]
(5) Zaaijer, H.L., Exel-Oehlers, P.V., Kraaijeveld, T., Altena, E., Lelie, P.N. (1992) Early detection of antibodies to HIV-1 by third-generation assays. Lancet 340, 770-772.
(6) Constantine, N.T., van der Groen, G., Belsey, E.M., Tamashiro, H. (1994) Sensitivity of HIV-antibody assays determined by seroconversion panels. AIDS 8, 1715-1720.
(7) Journal of Microbiological Methods, 45, 2001: 41-52.
(8) Duncan, R.J.S., Weston, P.D., Wrigglesworth, R., (1983) A new reagent which may be used to introduce sulfhydryl groups into proteins, and its use in the preparation of conjugates for immunoassay. Anal. Biochem. 132, 68.
(9) R Tanaka, T Yuhi, N Nagatani, T Endo, K Kerman, Y Takamura, E Tamiya (2006) A novel enhancement assay for immunochromatographic test strips using gold nanoparticles. Anal Bioanal Chem 385: 1414-1420.
(10) BF Giffin, K Gao, RE Morris and RR Cardell (1993) Enhancement of antigenic site detection with gold labeled secondary and tertiary antibodies using the immunogold-silver staining method. Biotechnic & Histochemistry 68: 309-315.
(11) P Hazarika, B Ceyhan, CM Niemeyer (2005) Sensitive detection of proteins using difunctional DNA-gold nanoparticles. Small 1: 844-848.

## Claims

1. A method for rapid immunochromatographic detection of a target in a sample comprising the step of forming a sandwich by contacting the sample with
(a) a first colloidal gold conjugate conjugated with a specific antibody or specific antigen to capture the target from a target site and at least one oligonucleotide or non-specific antibody or non-specific antigen; followed by
(b) another specific antibody or specific antigen to capture the target from another target site, wherein the other specific antibody or specific antigen is immobilized; and followed by
(c) a second colloidal gold conjugate conjugated with at least one oligonucleotide being complementary to the oligonucleotide of the first colloidal gold conjugate to capture the first colloidal conjugate from the oligonucleotide site or a non-specific antibody or non-specific antigen being complementary to the non-specific antibody or non-specific antigen of the first colloidal gold conjugate to capture the first colloidal conjugate from a non-specific antibody or non-specific antigen site.

2. The method according to claim 1, comprising the following steps of
(d) applying the sample to a sample application site;
(e) allowing the target in the sample getting captured from a target site by the first colloidal gold conjugate at a first conjugate releasing site;
(f) allowing the target in the sample to move to a test zone getting captured from another target site by the other immobilised specific antibody or specific antigen;
(g) allowing to release the second colloidal gold conjugate from a second conjugate releasing site for capturing the first colloidal gold conjugate from at least one oligonucleotide site or non-specific antibody or non-specific antigen site;
(h) allowing the sample to move through the test zone and a control zone to an absorbent site;
(i) allowing to continuously release the first and the second colloidal gold conjugates from the first and the second conjugate releasing sites to propagate to the test zone and the control zone;
(j) detecting a color in the control zone, and
(k) detecting a color in the test zone.

3. The method according to claims 1 or 2, wherein the specific antibody or antigen is selected from the group consisting of anti-beta chorionic gonadotropin hormone (anti-βhCG), anti-lipoarabinomannan (LAM), hepatitis virus antibodies against or antigens from hepatitis virus type A , hepatitis virus type B, or hepatitis virus type C or human immunoglobulin G antibodies or antigens.

4. The method according to claim 3, wherein the hepatitis virus antigen is hepatitis B surface antigen (HBsAg) and the hepatitis virus antibody is anti-HBsAg.

5. The method according to any of claims 1 to 4, wherein another specific antibody or antigen is selected from the group consisting of anti-alpha chorionic gonadotropin hormone (anti-αhCG), anti-lipoarabinomannan (LAM), hepatitis virus antibodies against or antigens from hepatitis virus type A , hepatitis virus type B, or hepatitis virus type C or human immunodeficiency virus (HIV) antibodies or antigens from the HIV type HIV-1 and HIV-2 or HIV subtype HIV-1-N, HIV-1-O or HIV-1-M.

6. The method according to claim 5, wherein the hepatitis virus antigen is hepatitis B surface antigen (HBsAg), the hepatitis virus antibody is anti-HBsAg and the human immunodeficiency virus (HIV) antigen is HIV p160.

7. The method according to any of claims 1 to 6, wherein the sample comprises a body fluid obtained from a subject.

8. The method according to claim 7, wherein the body fluid is selected from the group consisting of urine, whole blood, serum, plasma and saliva.

9. A test device for conducting the method for rapid immunochromatographic detection of a target in a sample of any of claims 1 to 8 comprising a housing comprising a test strip (101) comprising
(a) a sample application site (102);
(b) a first conjugate releasing site (103.1) comprising a first colloidal gold conjugate conjugated with a specific antibody or specific antigen to capture the target from a target site and at least one oligonucleotide or non-specific antibody or non-specific antigen;
(c) a second conjugate releasing site (103.2) comprising a second colloidal gold conjugate conjugated with at least one oligonucleotide being complementary to the oligonucleotide of the first colloidal conjugate to capture the first colloidal conjugate from the oligonucleotide site or a non-specific antibody or non-specific antigen being complementary to the non-specific antibody or non-specific antigen of the first colloidal gold conjugate to capture the first colloidal conjugate from a non-specific antibody or non-specific antigen site;
wherein the first and the second conjugate releasing sites are separated by a divider or wherein the first conjugate releasing site is on the test strip and the second conjugate releasing site is within the upper side of the housing;
(d) a nitrocellulose membrane (104);
(e) a test zone (108) comprising another specific antibody or specific antigen immobilized to capture the target from another target site;
(f) a control zone (109); and
(g) a sample absorbent site (105).

10. The test device according to claim 9, wherein the test strip (101) is attached to a supporting backing (107) by means of an adhesive (106).

11. The test device according to claim 10, wherein the supporting backing (107) is a plastic backing.

12. The test device according to claim 9, 10 or 11, wherein another specific antibody or specific antigen is selected from the group consisting of anti-alpha chorionic gonadotropin hormone (anti-αhCG), anti-lipoarabinomannan (LAM), hepatitis virus antibodies against or antigens from hepatitis virus type A , hepatitis virus type B, or hepatitis virus type C or human immunodeficiency virus (HIV) antibodies or antigens from the HIV type HIV-1 and HIV-2 or HIV subtype HIV-1-N, HIV-1-O or HIV-1-M.

13. The test device according to claim 12, wherein the hepatitis virus antigen is hepatitis B surface antigen (HBsAg), the hepatitis virus antibody is anti-HBsAg and the human immunodeficiency virus (HIV) antigen is HIV p160.

14. The test device according to any of claims 9 to 13, wherein the second conjugate releasing site (103.2) is laminated within the upper side of the housing.

15. A test device for conducting the method for rapid immunochromatographic detection of a target in a sample of any of claims 1 to 8 comprising a detection cup (510),
wherein the detection cup (510) is comprising
(a) a sample collecting container (501)
(b) a test strip comprising a sample application site (404); a nitrocellulose membrane (401); two test zones (403), wherein the test zones (403) comprise another specific antibody or antigen to capture the target from another target site, and a control zone (402); a sample absorbent site (405); and
(c) a first conjugate releasing site (503) comprising a first colloidal gold conjugate conjugated with a specific antibody or specific antigen to capture the target from a targe site and at least one oligonucleotide or non-specific antibody or non-specific antigen, and a second conjugate releasing site (504) comprising a second colloidal gold conjugate conjugated with at least one oligonucleotide being complementary to the oligonucleotide of the first colloidal conjugate to capture the first colloidal conjugate from the oligonucleotide site or a non-specific antibody or non-specific antigen being complementary to the non-specific antibody or non-specific antigen of the first colloidal gold conjugate to capture the first colloidal conjugate from a non-specific antibody or non-specific antigen site, separated from the test strip.

16. The test device according to claim 15, wherein another specific antibody or antigen is selected from the group consisting of anti-alpha chorionic gonadotropin hormone (anti-αhCG), anti-lipoarabinomannan (LAM), hepatitis virus antibodies against or antigens from hepatitis virus type A , hepatitis virus type B, or hepatitis virus type C or human immunodeficiency virus (HIV) antibodies or antigens from the HIV type HIV-1 and HIV-2 or HIV subtype HIV-1-N, HIV-1-O or HIV-1-M.

17. The test device according to claim 16, wherein the hepatitis virus antigen is hepatitis B surface antigen (HBsAg), the hepatitis virus antibody is anti-HBsAg and the human immunodeficiency virus (HIV) antigen is HIV p160.

18. Use of the method according to any of claims 1 to 8 for diagnosing and monitoring a disease or a specific condition of a subject by detecting a target in a sample.

19. Use according to claim 18, wherein the specific condition is pregnancy.

20. Use according to claims 18 or 19, wherein the target is human chorionic gonadotropin hormone (hCG).

21. Use according to claim 18, wherein the disease is hepatitis selected of the group consisting of hepatitis type A, hepatitis type B, or hepatitis type C.

22. Use according to claim 21, wherein the selected hepatitis type is hepatitis type B.

23. Use according to the claims 18, 21 or 22, wherein the target is hepatitis B surface antigen (HBsAg).

24. Use according to claim 18, wherein the disease is an HIV infection selected from the HIV infection group consisting of HIV type HIV-1 and HIV-2 or HIV subtype H1V-1-N, HIV-1-O or HIV-1-M.

25. Use according to claims 18 or 24, wherein the target is selected from an HIV antibody or antigen selected from the group consisting of p41, p120, p160, p18, p24/25, p55, p34, p40, p52, p68.

26. Use according to claim 25, wherein the HIV antigen is p160.

27. A kit for rapid immunochromatographic detection of a target in a sample comprising the test device according to any of claims 9 to 14 or the test device according to any of claims 15 to 17.

28. The kit according to claim 27, comprising further reagents, wash buffers and a manual.

29. A method for the manufacture of the test device according to any of claims 9 to 14 or of the test device according to any of claims 15 to 17,
comprising the following steps of
(a) preparing a first colloidal gold conjugate by adding a specific antibody or specific antigen to a conjugation buffer with at least one oligonucleotide or non-specific antibody or non-specific antigen and then adding it to a colloidal gold solution;
(b) preparing a second colloidal gold conjugate by adding at least one oligonucleotide being complementary to the oligonucleotide of the first colloidal gold conjugate or a non-specific antibody or non-specific antigen being complementary to the non-specific antibody or non-specific antigen of the first colloidal gold conjugate in a conjugation buffer to a colloidal gold solution; and
(c) preparing a first conjugate releasing site and a second conjugate releasing site by applying the first and the second gold conjugate on different pads;
wherein the first and second conjugate releasing sites are separated by a divider, or wherein the first conjugate releasing site is on the test strip and the second conjugate releasing site is within the upper side of a housing, of
wherein the first and second conjugate releasing sites are separated from the test strip in a detection cup.

30. The method according to claim 29, further comprising the following steps of
(d) preparing a sample application site, a test zone, a control zone and a sample absorbent site;
(e) assembling the sample application site, the test zone, the control zone, the sample absorbent site together with the first and the second conjugate releasing sites on a test strip;
(f) applying the first and the second colloidal gold conjugates on different sites of the same card separated by the divider; and
(g) assembling the test strip in a housing.

31. The method according to claim 29, further comprising the following steps of
(d) preparing a sample application site, a test zone, a control zone and a sample absorbent site;
(e) assembling the sample application site, the test zone, the control zone, the sample absorbent site together with the first conjugate releasing site on a test strip; and
(f) assembling the test strip and the second conjugate releasing site in the housing.

32. The method according to claim 29, further comprising the following steps of
(d) preparing a sample application site, two test zones, a control zone and a sample absorbent site;
(e) assembling the sample application site, the test zones, the control zone, and the sample absorbent site on a test strip; and
(f) assembling the test strip and the first and second conjugate releasing sites separately in a detection cup.

## Patentansprüche

1. Verfahren zur immunochromatographischen Schnelldetektion eines Targets in einer Probe, umfassend den Schritt der Bildung eines Sandwiches durch Inkontaktbringen der Probe mit
(a) einem ersten kolloidalen Goldkonjugat, konjugiert mit einem spezifischen Antikörper oder spezifischen Antigen, um das Target von einer Targetstelle einzufangen, und wenigstens einem Oligonukleotid oder nicht-spezifischen Antikörper oder nicht-spezifischen Antigen; gefolgt von
(b) einem weiteren spezifischen Antikörper oder spezifischen Antigen, um das Target von einer anderen Targetstelle einzufangen, wobei der andere spezifische Antikörper oder das andere spezifische Antigen immobilisiert ist; und gefolgt von
(c) einem zweiten kolloidalen Goldkonjugat, konjugiert mit wenigstens einem Oligonukleotid, das zu dem Oligonukleotid des ersten kolloidalen Goldkonjugats komplementär ist, um das erste kolloidale Konjugat von der Oligonukleotidstelle einzufangen, oder einem nicht-spezifischen Antikörper oder nicht-spezifischen Antigen, der/das zum nicht-spezifischen Antikörper oder nicht-spezifischen Antigen des ersten kolloidalen Goldkonjugats komplementär ist, um das erste kolloidale Konjugat von einer nicht-spezifischen Antikörper- oder nicht-spezifischen Antigenstelle einzufangen.

2. Verfahren nach Anspruch 1, das die folgenden Schritte umfasst
(d) Aufbringen der Probe auf eine Probenaufbringstelle;
(e) Erlauben, dass das Target in der Probe von einer Targetstelle durch das erste kolloidale Goldkonjugat an einer ersten Konjugatfreisetzungsstelle eingefangen wird;
(f) Erlauben, dass das Target in der Probe zu einer Testzone wandert, wobei es von einer anderen Targetstelle durch den anderen immobilisierten spezifischen Antikörper oder das andere immobilisierte spezifische Antigen eingefangen wird;
(g) Erlauben, dass das zweite kolloidale Goldkonjugat von einer zweiten Konjugatfreisetzungsstelle freigesetzt wird, um das erste kolloidale Goldkonjugat von wenigstens einer Oligonukleotidstelle oder nicht-spezifischen Antikörper- oder nicht-spezifischen Antigenstelle einzufangen;
(h) Erlauben, dass die Probe durch die Testzone und eine Kontrollzone zu einer Absorptionsstelle wandert;
(i) Erlauben, dass die ersten und die zweiten kolloidalen Goldkonjugate von den ersten und den zweiten Konjugatfreisetzungsstellen kontinuierlich freigesetzt werden, um sich zu der Testzone und der Kontrollzone auszubreiten;
(j) Nachweisen einer Farbe in der Kontrollzone und
(k) Nachweisen einer Farbe in der Testzone.

3. Verfahren nach den Ansprüchen 1 oder 2, wobei der spezifische Antikörper oder das spezifische Antigen ausgewählt ist aus der Gruppe, bestehend aus anti-beta-Choriongonadotropin-Hormon (anti-βhCG), anti-Lipoarabinomannan (LAM), Hepatitisvirus-Antikörpem gegen oder -Antigenen aus Hepatitisvirus Typ A, Hepatitisvirus Typ B oder Hepatitisvirus Typ C oder menschlichen Immunoglobulin-G-Antikörpern oder -Antigenen.

4. Verfahren nach Anspruch 3, wobei das Hepatitisvirus-Antigen Hepatitis-B-Oberflächenantigen (HBsAg) ist und der Hepatitisvirus-Antikörper anti-HBsAg ist.

5. Verfahren nach einem der Ansprüche 1 bis 4, wobei ein weiterer spezifischer Antikörper oder ein weiteres spezifisches Antigen ausgewählt ist aus der Gruppe, bestehend aus anti-alpha-Choriongonadotropin-Hormon (anti-αhCG), anti-Lipoarabinomannan (LAM), Hepatitisvirus-Antikörpern gegen oder -Antigenen aus Hepatitisvirus Typ A, Hepatitisvirus Typ B oder Hepatitisvirus Typ C oder menschlichen Immundefektvirus(HIV)-Antikörpern oder -Antigenen aus dem HIV-Typ HIV-1 oder HIV-2 oder HIV-Untertyp HIV-1-N, HIV-1-O oder HIV-1-M.

6. Verfahren nach Anspruch 5, wobei das Hepatitisvirus-Antigen Hepatitisvirus-B-Oberflächenantigen (HBsAg) ist, der Hepatitisvirus-Antikörper anti-HBsAg ist und das menschliche Immundefektvirus(HIV)-Antigen HIV p 160 ist.

7. Verfahren nach einem der Ansprüche 1 bis 6, wobei die Probe eine Körperflüssigkeit umfasst, die von einem Patienten erhalten ist.

8. Verfahren nach Anspruch 7, wobei die Körperflüssigkeit ausgewählt ist aus der Gruppe, bestehend aus Harn, Vollblut, Serum, Plasma und Speichel.

9. Testvorrichtung zur Durchführung des Verfahrens zur immunochromatographischen Schnelldetektion eines Targets in einer Probe nach einem der Ansprüche 1 bis 8, umfassend ein Gehäuse, das einen Teststreifen (101) umfasst, welcher umfasst
(a) eine Probenaufbringstelle (102);
(b) eine erste Konjugatfreisetzungsstelle (103.1), umfassend ein erstes kolloidales Goldkonjugat, konjugiert mit einem spezifischen Antikörper oder spezifischen Antigen, um das Target von einer Targetstelle einzufangen, und wenigstens einem Oligonukleotid oder nicht-spezifischen Antikörper oder nicht-spezifischen Antigen;
(c) eine zweite Konjugatfreisetzungsstelle (103.2), umfassend ein zweites kolloidales Goldkonjugat, konjugiert mit wenigstens einem Oligonukleotid, das zu dem Oligonukleotid des ersten kolloidalen Konjugats komplementär ist, um das erste kolloidale Konjugat von der Oligonukleotidstelle einzufangen, oder einem nicht-spezifischen Antikörper oder nicht-spezifischen Antigen, der/das zum nicht-spezifischen Antikörper oder nicht-spezifischen Antigen des ersten kolloidalen Goldkonjugats komplementär ist, um das erste kolloidale Konjugat von einer nicht-spezifischen Antikörper-oder nicht-spezifischen Antigenstelle einzufangen;
wobei die ersten und die zweiten Konjugatfreisetzungsstellen durch einen Teiler getrennt sind oder wobei die erste Konjugatfreisetzungstelle sich auf dem Teststreifen befindet und die zweite Konjugatfreisetzungstelle sich in der Oberseite des Gehäuses befindet;
(d) eine Nitrocellulose-Membran (104);
(e) eine Testzone (108), die einen weiteren spezifischen Antikörper oder ein weiteres spezifisches Antigen umfasst, der/das immobilisiert ist, um das Target von einer weiteren Targetstelle einzufangen;
(f) eine Kontrollzone (109); und
(g) eine Probenabsorptionsstelle (105).

10. Testvorrichtung nach Anspruch 9, wobei der Teststreifen (101) an einer Tragerückschicht (107) mittels eines Klebers (106) befestigt ist.

11. Testvorrichtung nach Anspruch 10, wobei die Tragerückschicht (107) eine Kunststoffrückschicht ist.

12. Testvorrichtung nach Anspruch 9, 10 oder 11, wobei ein weiterer spezifischer Antikörper oder ein weiteres spezifisches Antigen ausgewählt ist aus der Gruppe, bestehend aus anti-alpha-Choriongonadotropin-Hormon (anti-αhCG), anti-Lipoarabinomannan (LAM), Hepatitisvirus-Antikörpem gegen oder -Antigenen aus Hepatitisvirus Typ A, Hepatitisvirus Typ B oder Hepatitisvirus Typ C oder menschlichen Immundefektvirus (HIV)-Antikörpern oder -Antigenen aus dem HIV-Typ HIV-1 und HIV-2 oder HIV-Untertyp HIV-1-N, HIV-1-O oder HIV-1-M.

13. Testvorrichtung nach Anspruch 12, wobei das Hepatitisvirus-Antigen Hepatitis-B-Oberflächenantigen (HBsAg) ist, der Hepatitisvirus-Antikörper anti-HBsAg ist und das menschliche Immundefektvirus(HIV)-Antigen HIV p160 ist.

14. Testvorrichtung nach einem der Ansprüche 9 bis 13, wobei die zweite Konjugatfreisetzungsstelle (103.2) in der Oberseite des Gehäuses laminiert ist.

15. Testvorrichtung zur Durchführung des Verfahrens zur immunochromatographischen Schnelldetektion eines Targets in einer Probe nach einem der Ansprüche 1 bis 8, umfassend eine Detektionsschale (510), wobei die Detektionsschale (510)
(a) einen Probensammelbehälter (501)
(b) einen Teststreifen, der eine Probenaufbringstelle (404); eine Nitrocellulose-Membran (401); zwei Testzonen (403), wobei die Testzonen (403) einen weiteren spezifischen Antikörper oder ein weiteres spezifisches Antigen umfassen, um das Target von einer weiteren Targetstelle einzufangen, und eine Kontrollzone (402); eine Probenabsorptionsstelle (405) umfasst; und
(c) eine erste Konjugatfreisetzungsstelle (503), umfassend ein erstes kolloidales Goldkonjugat, konjugiert mit einem spezifischen Antikörper oder spezifischen Antigen, um das Target von einer Targetstelle einzufangen, und wenigstens einem Oligonukleotid oder nicht-spezifischen Antikörper oder nicht-spezifischen Antigen, und eine zweite Konjugatfreisetzungsstelle (504), umfassend ein zweites kolloidales Goldkonjugat, konjugiert mit wenigstens einem Oligonukleotid, das zu dem Oligonukleotid des ersten kolloidalen Konjugats komplementär ist, um das erste kolloidale Konjugat von der Oligonukleotidstelle einzufangen, oder einem nicht-spezifischen Antikörper oder nicht-spezifischen Antigen, das komplementär zum nicht-spezifischen Antikörper oder nicht-spezifischen Antigen des ersten kolloidalen Goldkonjugats ist, um das erste kolloidale Goldkonjugat von einer nicht-spezifischen Antikörper- oder nicht-spezifischen Antigenstelle einzufangen, getrennt vom Teststreifen,
umfasst.

16. Testvorrichtung nach Anspruch 15, wobei ein weiterer spezifischer Antikörper oder ein weiteres spezifisches Antigen ausgewählt ist aus der Gruppe, bestehend aus anti-alpha-Choriongonadotropin-Hormon (anti-αhCG), anti-Lipoarabinomannan (LAM), Hepatitisvirus-Antikörpem gegen oder -Antigenen aus Hepatitisvirus Typ A, Hepatitisvirus Typ B oder Hepatitisvirus Typ C oder menschlichen Immundefektvirus(HIV)-Antikörpern oder -Antigenen aus dem HIV-Typ HIV-1 oder HIV-2 oder HIV-Untertyp HIV-1-N, HIV-1-O oder HIV-1-M.

17. Testvorrichtung nach Anspruch 16, wobei das Hepatitisvirus-Antigen Hepatitis-B-Oberflächenantigen (HBsAg) ist, der Hepatitisvirus-Antikörper anti-HBsAg ist und das menschliche Immundefektvirus (HIV)-Antigen HIV p160 ist.

18. Verwendung des Verfahrens nach einem der Ansprüche 1 bis 8 zum Diagnostizieren und Überwachen einer Erkrankung oder eines spezifischen Zustandes eines Patienten durch Detektion eines Targets in einer Probe.

19. Verwendung nach Anspruch 18, wobei der spezifische Zustand Schwangerschaft ist.

20. Verwendung nach den Ansprüchen 18 oder 19, wobei das Target menschliches Choriongonadotropin-Hormon (hCG) ist.

21. Verwendung nach Anspruch 18, wobei die Erkrankung Hepatitis ist, ausgewählt aus der Gruppe, bestehend aus Hepatitis Typ A, Hepatitis Typ B oder Hepatitis Typ C.

22. Verwendung nach Anspruch 21, wobei der ausgewählte Hepatitis-Typ Hepatitis Typ B ist.

23. Verwendung nach den Ansprüchen 18, 21 oder 22, wobei das Target Hepatitis-B-Oberflächenantigen (HBsAg) ist.

24. Verwendung nach Anspruch 18, wobei die Erkrankung eine HIV-Infektion ist, ausgewählt aus der HIV-Infektionsgruppe, bestehend aus HIV-Typ HIV-1 und HIV-2 oder HIV-Untertyp HIV-1-N, HIV-1-O oder HIV-1-M.

25. Verwendung nach den Ansprüchen 18 oder 24, wobei das Target ausgewählt ist aus einem HIV-Antikörper oder -Antigen, ausgewählt aus der Gruppe, bestehend aus p41, p120, p160, p18, p24/25, p55, p34, p40, p52, p68.

26. Verwendung nach Anspruch 25, wobei das HIV-Antigen p160 ist.

27. Kit zur immunochromatographischen Schnelldetektion eines Targets in einer Probe, der die Testvorrichtung nach einem der Ansprüche 9 bis 14 oder die Testvorrichtung nach einem der Ansprüche 15 bis 17 umfasst.

28. Kit nach Anspruch 27, der weitere Reagentien, Waschpuffer und eine Anleitung umfasst.

29. Verfahren zur Herstellung der Testvorrichtung nach einem der Ansprüche 9 bis 14 oder der Testvorrichtung nach einem der Ansprüche 15 bis 17, das die folgenden Schritte umfasst
(a) Herstellen eines ersten kolloidalen Goldkonjugats durch Zugeben eines spezifischen Antikörpers oder spezifischen Antigens zu einem Konjugationspuffer mit wenigstens einem Oligonukleotid oder nicht-spezifischen Antikörper oder nicht-spezifischen Antigen und anschließendes Zugeben desselben zu einer kolloidalen Goldlösung;
(b) Herstellen eines zweiten kolloidalen Goldkonjugats durch Zugeben wenigstens eines Oligonukleotids, das zu dem Oligonukleotid des ersten kolloidalen Goldkonjugats komplementär ist, oder eines nicht-spezifischen Antikörpers oder nicht-spezifischen Antigens, der/das zum nicht-spezifischen Antikörper oder nicht-spezifischen Antigen des ersten kolloidalen Goldkonjugats komplementär ist, in einem Konjugationspuffer zu einer kolloidalen Goldlösung; und
(c) Herstellen einer ersten Konjugatfreisetzungsstelle und einer zweiten Konjugatfreisetzungstelle durch Aufbringen des ersten und des zweiten Goldkonjugats auf unterschiedliche Kissen;
wobei die ersten und zweiten Konjugatfreisetzungsstellen durch einen Teiler getrennt sind oder wobei die erste Konjugatfreisetzungsstelle sich auf dem Teststreifen befindet und die zweite Konjugatfreisetzungsstelle sich in der Oberseite eines Gehäuses befindet, oder
wobei die ersten und zweiten Konjugatfreisetzungsstellen vom Teststreifen in einer Detektionsschale getrennt sind.

30. Verfahren nach Anspruch 29, das weiter die folgenden Schritte umfasst
(d) Herstellen einer Probenaufbringstelle, einer Testzone, einer Kontrollzone und einer Probenabsorptionsstelle;
(e) Zusammenfügen der Probenaufbringstelle, der Testzone, der Kontrollzone, der Probenabsorptionsstelle mit den ersten und den zweiten Konjugatfreisetzungsstellen auf einem Teststreifen;
(f) Aufbringen der ersten und der zweiten kolloidalen Goldkonjugate auf unterschiedliche Stellen derselben Karte, getrennt durch den Teiler; und
(g) Zusammenfügen des Teststreifens in einem Gehäuse.

31. Verfahren nach Anspruch 29, das weiter die folgenden Schritte umfasst
(d) Herstellen einer Probenaufbringstelle, einer Testzone, einer Kontrollzone und einer Probenabsorptionsstelle;
(e) Zusammenfügen der Probenaufbringstelle, der Testzone, der Kontrollzone, der Probenabsorptionsstelle mit der ersten Konjugatfreisetzungsstelle auf einem Teststreifen; und
(f) Zusammenfügen des Teststreifens und der zweiten Konjugatfreisetzungsstelle im Gehäuse.

32. Verfahren nach Anspruch 29, das weiter die folgenden Schritte umfasst
(d) Herstellen einer Probenaufbringstelle, zweier Testzonen, einer Kontrollzone und einer Probenabsorptionsstelle;
(e) Zusammenfügen der Probenaufbringstelle, der Testzonen, der Kontrollzone und der Probenabsorptionsstelle auf einem Teststreifen; und
(f) Zusammenfügen des Teststreifens und der ersten und zweiten Konjugatfreisetzungsstellen getrennt in einer Detektionsschale.

## Revendications

1. Procédé de détection rapide immunochromatographique d'une cible dans un échantillon comprenant l'étape consistant à former un sandwich en mettant l'échantillon en contact avec
(a) un premier conjugué constitué d'or colloïdal conjugué à un anticorps spécifique ou à un antigène spécifique pour capturer la cible à partir d'un site cible et au moins un oligonucléotide ou un anticorps non spécifique ou un antigène non spécifique ; suivie par
(b) un autre anticorps spécifique ou antigène spécifique pour capturer la cible à partir d'un autre site cible, dans lequel l'autre anticorps spécifique ou antigène spécifique est immobilisé ; et suivie par
(c) un deuxième conjugué constitué d'or colloïdal conjugué à au moins un oligonucléotide qui est complémentaire à l'oligonucléotide du premier conjugué constitué d'or colloïdal pour capturer le premier conjugué colloïdal à partir du site oligonucléotidique ou à un anticorps non spécifique ou à un antigène non spécifique qui est complémentaire à l'anticorps non spécifique ou à l'antigène non spécifique du premier conjugué constitué d'or colloïdal pour capturer le premier conjugué colloïdal à partir d'un site de l'anticorps non spécifique ou de l'antigène non spécifique.

2. Procédé selon la revendication 1, comprenant les étapes suivantes qui consistent à :
(d) appliquer l'échantillon à un site d'application de l'échantillon ;
(e) permettre à la cible dans l'échantillon d'être capturée à partir d'un site cible par le premier conjugué constitué d'or colloïdal à un site de libération du premier conjugué ;
(f) permettre à la cible dans l'échantillon de se déplacer à une zone d'essai d'être capturée à partir d'un autre site cible par l'autre anticorps spécifique ou antigène spécifique immobilisé ;
(g) permettre la libération du deuxième conjugué constitué d'or colloïdal à partir d'un site de libération du deuxième conjugué pour capturer le premier conjugué constitué d'or colloïdal à partir d'au moins un site oligonucléotidique ou d'un site de l'anticorps non spécifique ou de l'antigène non spécifique ;
(h) permettre à l'échantillon de se déplacer à travers la zone d'essai et une zone de contrôle vers un site absorbant ;
(i) permettre la libération continue des premier et deuxième conjugués constitués d'or colloïdal à partir des sites de libération des premier et deuxième conjugués pour leur propagation jusqu'à la zone d'essai et la zone de contrôle ;
(j) détecter une couleur dans la zone de contrôle, et
(k) détecter une couleur dans la zone d'essai.

3. Procédé selon les revendications 1 ou 2, dans lequel l'anticorps ou l'antigène spécifique est sélectionné dans le groupe composé d'un anticorps dirigé contre la sous-unité béta de l'hormone gonadotrophine chorionique humaine (anti-βhCG), d'un anticorps anti-Lipoarabinomannan (LAM), d'anticorps anti-hépatite contre le virus de l'hépatite A ou d'antigènes de l'hépatite du virus de l'hépatite A, d'anticorps contre ou d'antigènes du virus de l'hépatite B, ou du virus de l'hépatite C ou de l'immunoglobuline G humaine.

4. Procédé selon la revendication 3, dans lequel l'antigène du virus de l'hépatite est un antigène de surface de l'hépatite B (HBsAg) et l'anticorps contre le virus de l'hépatite est un anticorps anti-HBsAg.

5. Procédé selon l'une quelconque des revendications 1 à 4, dans lequel un autre anticorps ou antigène spécifique est sélectionné dans le groupe composé d'un anticorps dirigé contre la sous-unité alpha de l'hormone gonadotrophine chorionique humaine (anti-αhCG), d'un anticorps anti-Lipoarabinomannan (LAM), d'anticorps anti-hépatite contre le virus de l'hépatite A ou d'antigènes de l'hépatite du virus de l'hépatite A, d'anticorps contre ou d'antigènes du virus de l'hépatite B, ou du virus de l'hépatite C ou du virus de l'immunodéficience humaine (VIH) du VIH de type VIH-1 et VIH-2 ou de sous-type VIH-1 groupe N, VIH-1 groupe O ou VIH-1 groupe M.

6. Procédé selon la revendication 5, dans lequel l'antigène du virus de l'hépatite est un antigène de surface de l'hépatite B (HBsAg), l'anticorps contre le virus de l'hépatite est l'anti-HBsAg et l'antigène du virus de l'immunodéficience humaine (VIH) est l'antigène p160 du VIH.

7. Procédé selon l'une quelconque des revendications 1 à 6, dans lequel l'échantillon comprend un fluide corporel obtenu d'un sujet.

8. Procédé selon la revendication 7, dans lequel le fluide corporel est sélectionné dans le groupe composé d'urine, de sang total, de sérum, de plasma et de salive.

9. Dispositif d'essai pour réaliser le procédé de détection rapide immunochromatographique d'une cible dans un échantillon de l'une quelconque des revendications 1 à 8 comprenant un coffret incluant une bande d'essai (101) comprenant
(a) un site (102) d'application de l'échantillon ;
(b) un site (103.1) de libération d'un premier conjugué comprenant un premier conjugué constitué d'or colloïdal conjugué à un anticorps spécifique ou antigène spécifique pour capturer la cible à partir d'un site cible et au moins un oligonucléotide ou anticorps non spécifique ou antigène non spécifique ;
(c) un site (103.2) de libération d'un deuxième conjugué comprenant un deuxième conjugué constitué d'or colloïdal conjugué à au moins un oligonucléotide qui est complémentaire à l'oligonucléotide du premier conjugué colloïdal pour capturer le premier conjugué colloïdal à partir du site oligonucléotidique ou à un anticorps non spécifique ou à un antigène non spécifique qui est complémentaire à l'anticorps non spécifique ou à l'antigène non spécifique du premier conjugué constitué d'or colloïdal pour capturer le premier conjugué colloïdal à partir d'un site de l'anticorps non spécifique ou de l'antigène non spécifique ;
dans lequel les sites de libération des premier et deuxième conjugués sont séparés par un séparateur ou dans lequel le site de libération du premier conjugué se trouve sur la bande d'essai et le site de libération du deuxième conjugué se trouve dans la face supérieure du coffret ;
(d) une membrane de nitrocellulose (104) ;
(e) une zone d'essai (108) comprenant un autre anticorps spécifique ou antigène spécifique immobilisé pour capturer la cible à partir d'un autre site cible ;
(f) une zone de contrôle (109) ; et
(g) un site absorbant l'échantillon (105).

10. Dispositif d'essai selon la revendication 9, dans lequel la bande d'essai (101) est attachée à un support (107) au moyen d'un adhésif (106).

11. Dispositif d'essai selon la revendication 10, dans lequel le support (107) est un support plastique.

12. Dispositif d'essai selon la revendication 9, 10 ou 11, dans lequel un autre anticorps spécifique ou antigène spécifique est sélectionné dans le groupe composé d'un anticorps dirigé contre la sous-unité alpha de l'hormone gonadotrophine chorionique humaine (anti-αhCG), d'un anticorps anti-Lipoarabinomannan (LAM), d'anticorps anti-hépatite contre le virus de l'hépatite A ou d'antigènes de l'hépatite du virus de l'hépatite A, d'anticorps contre ou d'antigènes du virus de l'hépatite B, ou du virus de l'hépatite C ou du virus de l'immunodéficience humaine (VIH) du VIH de type VIH-1 et VIH-2 ou de sous-type VIH-1 groupe N, VIH-1 groupe O ou VIH-1 groupe M.

13. Dispositif d'essai selon la revendication 12, dans lequel l'antigène du virus de l'hépatite est un antigène de surface de l'hépatite B (HBsAg), l'anticorps contre le virus de l'hépatite est l'anticorps anti-HBsAg et l'antigène du virus de l'immunodéficience humaine (VIH) est l'antigène p160 du VIH.

14. Dispositif d'essai selon l'une quelconque des revendications 9 à 13, dans lequel le site de libération (103.2) du deuxième conjugué est stratifié dans la face supérieure du coffret.

15. Dispositif d'essai pour réaliser le procédé de détection rapide immunochromatographique d'une cible dans un échantillon de l'une quelconque des revendications 1 à 8, comprenant une coupe de détection (510),
dans lequel la coupe de détection (510) comprend
(a) un flacon de collecte d'échantillon (501)
(b) une bande d'essai comprenant un site d'application de l'échantillon (404) ; une membrane de nitrocellulose (401) ; deux zones d'essai (403), dans lequel les zones d'essai (403) comprennent un autre anticorps ou antigène spécifique pour capturer la cible à partir d'un autre site cible, et une zone de contrôle (402) ; un site d'absorption l'échantillon (405) ; et
(c) un site de libération (503) d'un premier conjugué comprenant un premier conjugué constitué d'or colloïdal conjugué à un anticorps spécifique ou à un antigène spécifique pour capturer la cible à partir d'un site cible et au moins un oligonucléotide ou anticorps non spécifique ou antigène non spécifique, et un site de libération (504) d'un deuxième conjugué comprenant un deuxième conjugué constitué d'or colloïdal conjugué à au moins un oligonucléotide qui est complémentaire à l'oligonucléotide du premier conjugué colloïdal pour capturer le premier conjugué colloïdal à partir du site oligonucléotidique ou à un anticorps non spécifique ou à un antigène non spécifique qui est complémentaire à l'anticorps non spécifique ou à l'antigène non spécifique du premier conjugué constitué d'or colloïdal pour capturer le premier conjugué colloïdal à partir d'un site de l'anticorps non spécifique ou de l'antigène non spécifique, séparés de la bande d'essai.

16. Dispositif d'essai selon la revendication 15, dans lequel un autre anticorps ou antigène spécifique est sélectionné dans le groupe composé d'un anticorps dirigé contre la sous-unité alpha de l'hormone gonadotrophine chorionique humaine (anti-αhCG), d'un anticorps anti-Lipoarabinomannan (LAM), d'anticorps anti-hépatite contre le virus de l'hépatite A ou d'antigènes de l'hépatite du virus de l'hépatite A, d'anticorps contre ou d'antigènes du virus de l'hépatite B, ou du virus de l'hépatite C ou d'anticorps ou d'antigènes du virus de l'immunodéficience humaine (VIH) du VIH de type VIH-1 et VIH-2 ou de sous-type VIH-1 groupe N, VIH-1 groupe O ou VIH-1 groupe M.

17. Dispositif d'essai selon la revendication 16, dans lequel l'antigène du virus de l'hépatite est un antigène de surface de l'hépatite B (HBsAg), l'anticorps contre le virus de l'hépatite est l'anticorps anti-HBsAg et l'antigène du virus de l'immunodéficience humaine (VIH) est l'antigène p160 du VIH.

18. Utilisation du procédé selon l'une quelconque des revendications 1 à 8 pour le diagnostic et la surveillance d'une maladie ou d'un état spécifique d'un sujet en détectant une cible dans un échantillon.

19. Utilisation selon la revendication 18, dans laquelle l'état spécifique est une grossesse.

20. Utilisation selon les revendications 18 ou 19, dans laquelle la cible est l'hormone gonadotrophine chorionique humaine (hCG).

21. Utilisation selon la revendication 18, dans laquelle la maladie est une hépatite sélectionnée dans le groupe composé de l'hépatite A, de l'hépatite B ou de l'hépatite C.

22. Utilisation selon la revendication 21, dans laquelle le type de l'hépatite sélectionnée est l'hépatite B.

23. Utilisation selon les revendications 18, 21 ou 22, dans laquelle la cible est un antigène de surface de l'hépatite B (HBsAg).

24. Utilisation selon la revendication 18, dans laquelle la maladie est une infection par le VIH sélectionnée du groupe d'infections par le VIH composé du VIH de type VIH-1 et VIH-2 ou de sous-type VIH-1 groupe N, VIH-1 groupe O ou VIH-1 groupe M.

25. Utilisation selon les revendications 18 ou 24, dans laquelle la cible est sélectionnée d'un anticorps contre le VIH ou d'un antigène du VIH sélectionné du groupe composé des antigènes p41, p120, p160, p18, p24/25, p55, p34, p40, p52, p68.

26. Utilisation selon la revendication 25, dans laquelle l'antigène du VIH est l'antigène p160.

27. Trousse pour la détection rapide immunochromatographique d'une cible dans un échantillon comprenant le dispositif d'essai selon l'une quelconque des revendications 9 à 14 ou le dispositif d'essai selon l'une quelconque des revendications 15 à 17.

28. Trousse selon la revendication 27, comprenant en outre des réactifs, des tampons de lavage et un manuel.

29. Procédé de fabrication du dispositif d'essai selon l'une quelconque des revendications 9 à 14 ou du dispositif d'essai selon l'une quelconque des revendications 15 à 17, comprenant les étapes suivantes qui consistent à (a) préparer un premier conjugué constitué d'or colloïdal en ajoutant un anticorps spécifique ou un antigène spécifique à un tampon de conjugaison avec au moins un oligonucléotide ou anticorps non spécifique ou antigène non spécifique et l'ajouter ensuite à une solution constituée d'or colloïdal ;
(b) préparer un deuxième conjugué constitué d'or colloïdal en ajoutant au moins un oligonucléotide qui est complémentaire à l'oligonucléotide du premier conjugué constitué d'or colloïdal ou à un anticorps non spécifique ou à un antigène non spécifique qui est complémentaire à l'anticorps non spécifique ou à l'antigène non spécifique du premier conjugué constitué d'or colloïdal dans un tampon de conjugaison à une solution constituée d'or colloïdal ; et
(c) préparer un site de libération du premier conjugué et un site de libération du deuxième conjugué en appliquant le premier et le deuxième conjugués à d'or sur des tampons différents ;
dans lequel les sites de libération du premier et du deuxième conjugués sont séparés par un séparateur, ou dans lequel le site de libération du premier conjugué se trouve sur la bande d'essai et le site de libération du deuxième conjugué se trouve dans la face supérieure d'un coffret, ou
dans lequel les sites de libération du premier et du deuxième conjugués sont séparés de la bande d'essai dans une coupe de détection.

30. Procédé selon la revendication 29, comprenant en outre les étapes suivantes qui consistent à :
(d) préparer un site d'application de l'échantillon, une zone d'essai, une zone de contrôle et un site absorbant l'échantillon ;
(e) assembler le site d'application de l'échantillon, la zone d'essai, la zone de contrôle, le site absorbant l'échantillon ensemble avec les sites de libération des premier et deuxième conjugués sur une bande d'essai ;
(f) appliquer le premier et le deuxième conjugués constitués d'or colloïdal sur des sites différents de la même carte séparés par le séparateur ; et
(g) assembler la bande d'essai dans un coffret.

31. Procédé selon la revendication 29, comprenant en outre les étapes suivantes qui consistent à (d) préparer un site d'application de l'échantillon, une zone d'essai, une zone de contrôle et un site absorbant l'échantillon ;
(e) assembler le site d'application de l'échantillon, la zone d'essai, la zone de contrôle, le site absorbant l'échantillon ensemble avec le site de libération du premier conjugué sur une bande d'essai ; et
(f) assembler la bande d'essai et le site de libération du deuxième conjugué dans le coffret.

32. Procédé selon la revendication 29, comprenant en outre les étapes suivantes qui consistent à (d) préparer un site d'application de l'échantillon, deux zones d'essai, une zone de contrôle et un site absorbant l'échantillon ;
(e) assembler le site d'application de l'échantillon, les deux zones d'essai, la zone de contrôle, et le site absorbant l'échantillon sur une bande d'essai ; et
(f) assembler la bande d'essai et les sites de libération du premier et du deuxième conjugués dans une coupe de détection.
